# EUROPEAN PATENT SPECIFICATION

(11) **EP 3 791 876 B1**
(45) Date of publication and mention of the grant of the patent: **04.11.2026**
(21) Application number: 20190127.9
(22) Date of filing: 19.08.2016
(51) Int. Cl.: A61K 31/436, A61P 35/04, A61K 31/519, A61K 31/513, A61K 31/475, A61K 31/4745, A61K 31/282, A61K 9/127, A61K 9/00, A61K 9/1271, A61K 47/20, A61P 43/00

(54) **METHODS FOR TREATING METASTATIC PANCREATIC CANCER USING COMBINATION THERAPIES COMPRISING LIPOSOMAL IRINOTECAN AND OXALIPLATIN**
VERFAHREN ZUR BEHANDLUNG VON METASTASIERENDEM BAUCHSPEICHELDRÜSENKREBS MITHILFE VON KOMBINATIONSTHERAPIEN MT LIPOSOMALEM IRINOTECAN UND OXALIPLATIN
MÉTHODES PERMETTANT DE TRAITER LE CANCER DU PANCRÉAS MÉTASTATIQUE AU MOYEN DE TRAITEMENTS D'ASSOCIATION COMPRENANT DE L'IRINOTÉCAN LIPOSOMAL ET DE L'OXALIPLATINE

(30) Priority: 21.08.2015 US 201562208209 P; 10.09.2015 US 201562216736 P; 30.12.2015 US 201562273244 P; 21.01.2016 US 201662281473 P; 02.03.2016 US 201662302341 P; 15.04.2016 US 201662323245 P; 31.05.2016 US 201662343313 P
(43) Date of publication of application: 17.03.2021
(62) Document number(s) of the earlier application(s) in accordance with Art. 76 EPC: 16758337.6 / 3 337 478
(73) Proprietor: Ipsen Biopharm Ltd., Wrexham Industrial Estate Wrexham LL13 9UF (GB)
(72) Inventor: BLANCHETTE, Sarah F., Lynnfield, MA Massachusetts 01940 (US); FITZGERALD, Jonathan Basil, Arlington, MA Massachusetts 02474 (US); GADDY, Daniel F., Cambridge, MA Massachusetts 02141 (US); HENDRIKS, Bart S., Belmont, MA Massachusetts 02478 (US); KALRA, Ashish, Belmont, MA 02478 (US); LEE, Helen, Cambridge, MA Massachusetts 02140 (US); BAYEVER, Eliel, New York, NY New York 10023 (US)
(74) Representative: Carpmaels & Ransford LLP

(56) References cited:
- CHANG T C ET AL: "Phase I study of nanoliposomal irinotecan (PEP02) in advanced solid tumor patients", CANCER CHEMOTHERAPY AND PHARMACOLOGY, SPRINGER VERLAG, BERLIN, vol. 75, no. 3, 11 January 2015 (2015-01-11), pages 579 - 586, XP035456963, ISSN: 0344-5704, [retrieved on 20150111], DOI: 10.1007/S00280-014-2671-X
- L. CHEN, H. SHIAH, T. CHAO, R. K. HSIEH, G. CHEN, J. CHANG, G. YEH: "Phase I study of liposome irinotecan (PEP02) in combination with weekly infusion of 5-FU/LV in advanced solid tumors", JOURNAL OF CLINICAL ONCOLOGY, vol. 28, no. 15_Suppl., E13024, 2010, XP002763720, Retrieved from the Internet <URL:http://ascopubs.org/doi/abs/10.1200/jco.2010.28.15_suppl.e13024> [retrieved on 20161102], DOI: 10.1200/jco.2010.28.15_suppl.e13024
- KO A H ET AL: "A multinational phase 2 study of nanoliposomal irinotecan sucrosofate (PEP02, MM-398) for patients with gemcitabine-refractory metastatic pancreatic cancer", BRITISH JOURNAL OF CANCER 20 AUG 2013, vol. 109, no. 4, 20 August 2013 (2013-08-20), pages 920 - 925, XP002763721, ISSN: 1532-1827
- PETER J HOSEIN ET AL: "A retrospective study of neoadjuvant FOLFIRINOX in unresectable or borderline-resectable locally advanced pancreatic adenocarcinoma", BMC CANCER, BIOMED CENTRAL, LONDON, GB, vol. 12, no. 1, 29 May 2012 (2012-05-29), pages 199, XP021126474, ISSN: 1471-2407, DOI: 10.1186/1471-2407-12-199

## Description

### TECHNICAL FIELD

This disclosure relates to novel therapies useful in the treatment of pancreatic cancer which comprise the use of liposomal irinotecan in combination with 5-fluorouracil and oxaliplatin for the (first line) treatment of patients diagnosed with previously untreated pancreatic cancer.

### BACKGROUND

Pancreatic cancer is chemotherapy-resistant, with an extremely poor prognosis. It is the fourth leading cause of cancer death in the United States; the 5-year survival rate is 6%. The incidence of pancreatic cancer has increased during the past several decades and in 2014, an estimated 46,420 patients were diagnosed with pancreatic cancer and 39,590 died. Pancreatic cancer is projected to surpass liver, breast, prostate, and colorectal cancers to become the second-leading cause of cancer-related death by 2030. These statistics reflect the dire nature of the disease and lack of effective therapies. The location of the tumor results in few early symptoms and is often diagnosed at a late stage as a result. The absence of effective screening tools, and a limited understanding of risk factors, means that patients have advanced or metastatic disease at the time of diagnosis. Given the poor prognosis and the low median survival rates of less than one year for patients with metastatic disease, new treatment options are still needed.

Tolerability of multi-drug regimens is important in cancer treatment. The longer the duration of manageable treatment should translate into improved outcome due to longer drug exposure. During the last 5 years, one combination chemotherapy regimen that has emerged as standard of care for first-line treatment of metastatic pancreatic cancer is the combination therapy of 5-fluorouricil (5-FU)/leucovorin (LV) + irinotecan + oxaliplatin (FOLFIRINOX). However, FOLFIRINOX is known to have significant toxicity, and use is limited to patients with better performance status (i.e. ECOG performance score of 0 or 1). With prolonged FOLFIRINOX treatment, oxaliplatin is often discontinued from the regimen due to toxicity. Therefore, if equally effective double regimens can be identified, patients may be able to tolerate prolonged treatment better, and even poor performance status patients may receive benefit. Although the FOLFIRINOX regimen has been recommended by the National Comprehensive Cancer Network (NCCN) as a preferred option for first-line metastatic disease since 2011, there are some concerns about the toxicity associated with FOLFIRINOX. One dose regimen of FOLFIRINOX is 85 mg/m² oxaliplatin, 180 mg/m² irinotecan, and fluorouracil at a dose of 400 mg/m² administered by IV bolus followed by a continuous infusion of 2400 mg/m². Yet due to toxicity, modified FOLFIRINOX regimens are often used (e.g. elimination of the 5-FU bolus) with unknown effects on the efficacy and safety of modified schedules.

CPT-11 is irinotecan hydrochloride trihydrate, marketed as Camptosar^{®} in the United States. MM-398 is a liposomal irinotecan and is marketed in the U.S. as the FDA-approved product ONIVYDE^{®} in combination with 5-fluorouracil and leucovorin for the treatment of patients with metastatic adenocarcinoma of the pancreas after disease progression following gemcitabine-based therapy. Chang et al. (Cancer Chemotherapy and Pharmacology, March 2015, Volume 75, Issue 3, pp 579-586) describes a "[p]hase I study of nanoliposomal irinotecan (PEP02) in advanced solid tumor patients". Chen et al. (Journal of Clinical Oncology, Vol. 28, No. 15) describes a " Phase I study of liposome irinotecan (PEP02) in combination with weekly infusion of 5-FU/LV in advanced solid tumors". Ko et al. (British Journal of Cancer, vol. 109, No. 4, pages 920-925) describes "[a] multinational phase 2 study of nanoliposomal irinotecan sucrosofate (PEP02, MM-398) for patients with gemcitabine-refractory metastatic pancreatic cancer". Hosein et al. (BMC Cancer 2012 12:199) describes "[a] retrospective study of neoadjuvant FOLFIRINOX in unresectable or borderline-resectable locally advanced pancreatic adenocarcinoma".

### SUMMARY

The invention is defined in the appended claims. Any references in the description to methods of treatment refer to the compounds, pharmaceutical compositions and medicaments of the present invention for use in a method for treatment of the human (or animal) body by therapy. As used in relation to the invention, any reference to liposomal irinotecan refers to irinotecan sucrose octasulfate salt liposome injection. In one aspect, the invention relates to liposomal irinotecan for use in a method of treating metastatic adenocarcinoma of the pancreas in a human patient who has not previously received chemotherapy to treat the metastatic adenocarcinoma of the pancreas, wherein the liposomal irinotecan is administered in combination with oxaliplatin, leucovorin, and 5-fluorouracil, the method comprising administering an antineoplastic therapy to the patient, the antineoplastic therapy consisting of:
a. 60 mg/m² of liposomal irinotecan,
b. 60 mg/m² to 85 mg/m² oxaliplatin,
c. 200 mg/m² of (I)-form of leucovorin or 400 mg/m² of the (I+d) racemic form of leucovorin, and
d. 2,400 mg/m² 5-fluorouracil;
wherein the liposomal irinotecan is irinotecan sucrose octasulfate salt liposome injection.

Preferred embodiments of the invention are defined in the dependent claims.

Improved antineoplastic therapies for the treatment of pancreatic cancer provide the administration of liposomal irinotecan in combination with oxaliplatin and 5-fluorouracil to patients with previously untreated pancreatic cancer (e.g., untreated metastatic pancreatic adenocarcinoma, or mPAC). The 5-fluorouracil can be administered in combination with leucovorin. The improved antineoplastic therapies can provide improved therapeutic index (e.g., improved toxicity profiles) relative to prior FOLFIRINOX regimens.

A method of treating pancreatic cancer can comprise the administration of an antineoplastic therapy of liposomal irinotecan, oxaliplatin, and 5-fluorouracil once every two weeks to the patient. Optionally, leucovorin can also be administered prior to each administration of the 5-fluorouracil. Each administration of the liposomal irinotecan can be administered in a total dose of 60 mg/m² liposomal irinotecan (dose based on the amount of irinotecan hydrochloride trihydrate, as defined herein). A total of 2,400 mg/m² 5-fluorouracil can be administered over 46 hours starting on each day when the liposomal irinotecan is administered. A total of 60, 75 or 85 mg/m² oxaliplatin can be administered on each day the liposomal irinotecan is administered. A total of 200 mg/m² (l) leucovorin can be administered prior to each administration of the 5-flurouracil (e.g., optionally administered as 400 mg/m² of (l+d) leucovorin). The antineoplastic therapy can be administered starting on days 1 and 15 of a 28-day treatment cycle, with the liposomal irinotecan, oxaliplatin, and optionally leucovorin administered on days 1 and 15 and initiating the 46-hour administration of the 5-fluorouracil on days 1 and 15.

The invention is based in part on several pre-clinical discoveries. First, liposomal irinotecan improved anti-tumor activity of the topoisomerase 1 inhibitor SN-38 (an active metabolite of irinotecan) relative to exposure-matched doses of non-liposomal irinotecan. Second, liposomal irinotecan combined with 5-fluorouracil and oxaliplatin consistently improved tumor growth inhibition and survival in mouse xenograft models of pancreatic cancer relative to non-liposomal irinotecan, without exacerbating the baseline toxicities of these agents.

**In** addition, the invention is based in part on the discovery that the administration of a dose of 80 mg/m² liposomal irinotecan was not well tolerated in humans when administered in combination with 60 mg/m² oxaliplatin, 2400 mg/m² 5-fluorouracil and 400 mg/m² (I+d) leucovorin. Accordingly, preferred methods of treating (previously untreated) pancreatic cancer provide for the administration of a human-tolerated antineoplastic therapy once every two weeks, where each administration of the antineoplastic therapy is a combination of the antineoplastic agents liposomal irinotecan, oxaliplatin and 5-fluorouracil provided herein. Preferably, the antineoplastic therapy administered once every two weeks consists of: (a) a total dose of 60 mg/m² liposomal irinotecan (dose based on the amount of irinotecan hydrochloride trihydrate, as defined herein), (b) a total dose of 60-85 mg/m² oxaliplatin (including, e.g., 60 or 85 mg/m²), and (c) a total of 2,400 mg/m² 5-fluorouracil optionally administered in combination with leucovorin. Optionally, the combination can include administration of a total of 200 mg/m² (l) leucovorin (optionally administered as 400 mg/m² of (l+d) leucovorin), prior to initiating the administration of the 5-fluorouracil. Preferably, no other antineoplastic agent is administered during the antineoplastic therapy, other than amounts of SN-38 produced within the patient from the liposomal irinotecan, after administration of the liposomal irinotecan. For example, the antineoplastic therapy can be administered without (non-liposomal) CPT-11 irinotecan. Preferably, the liposomal irinotecan, oxaliplatin, and (optionally) leucovorin are consecutively administered as separate infusions on a single (first) day and the 5-fluorouracil is administered starting on the first day after the administration of the leucovorin (if administered) and continuing into the following day (e.g., over a total of 46 hours).

### BRIEF DESCRIPTION OF THE DRAWINGS

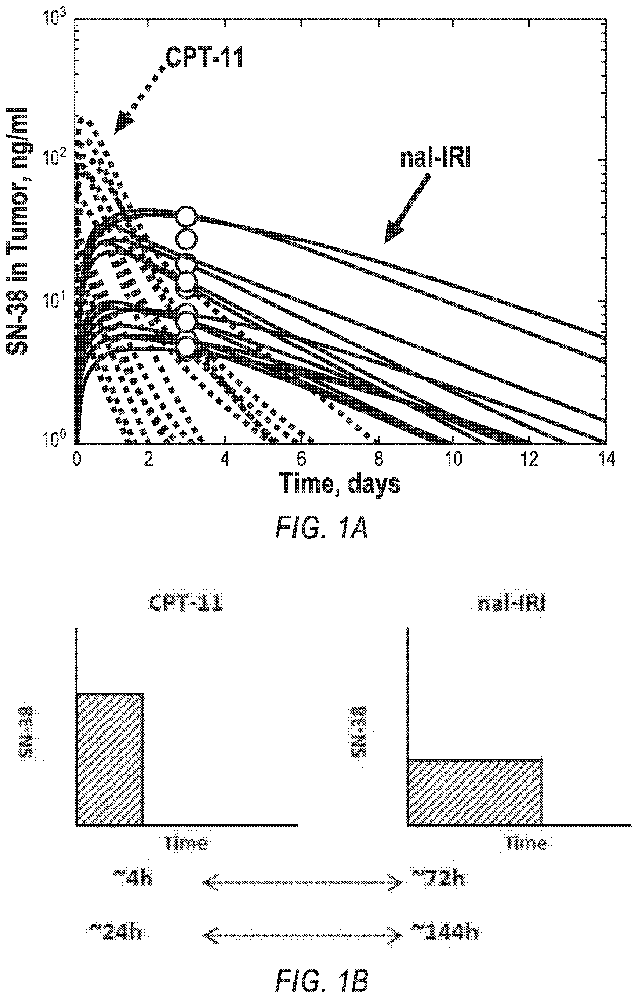

Figure 1A is a graph showing the simulated levels of the active irinotecan metabolite SN-38 over time based on liposomal irinotecan human clinical biopsy data and human clinical trial data.
Figure 1B is a schematic showing how the tumor exposure of SN-38 over time observed with liposomal irinotecan (MM-398) is prolonged compared to SN-38 tumor exposure from non-liposomal irinotecan (CPT-11).

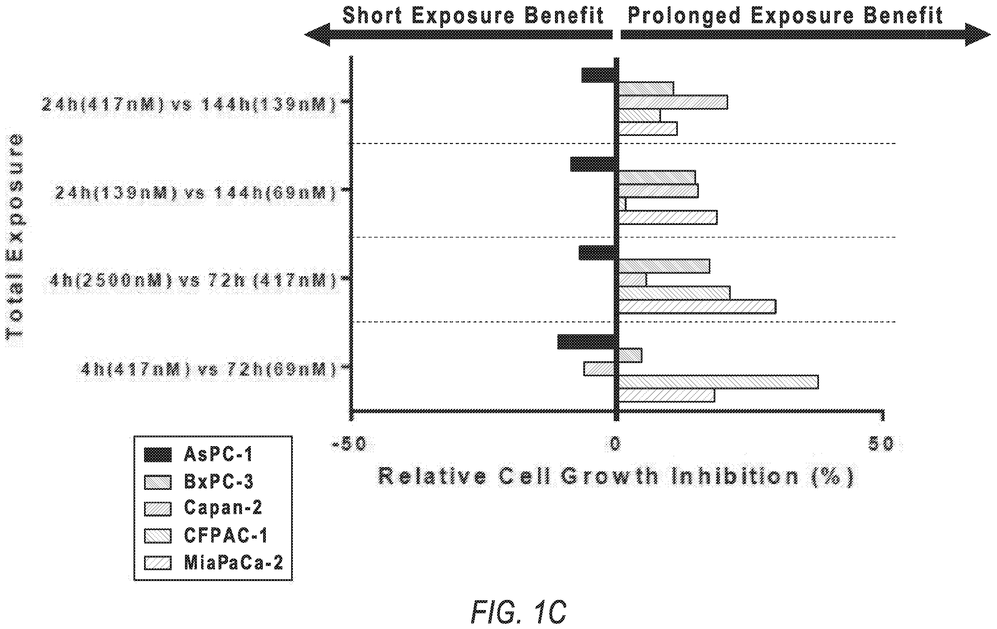

Figure 1C is a graph showing the percent relative cell growth inhibition of SN-38 based on various times of total SN-38 cell exposure for 5 different cell lines.

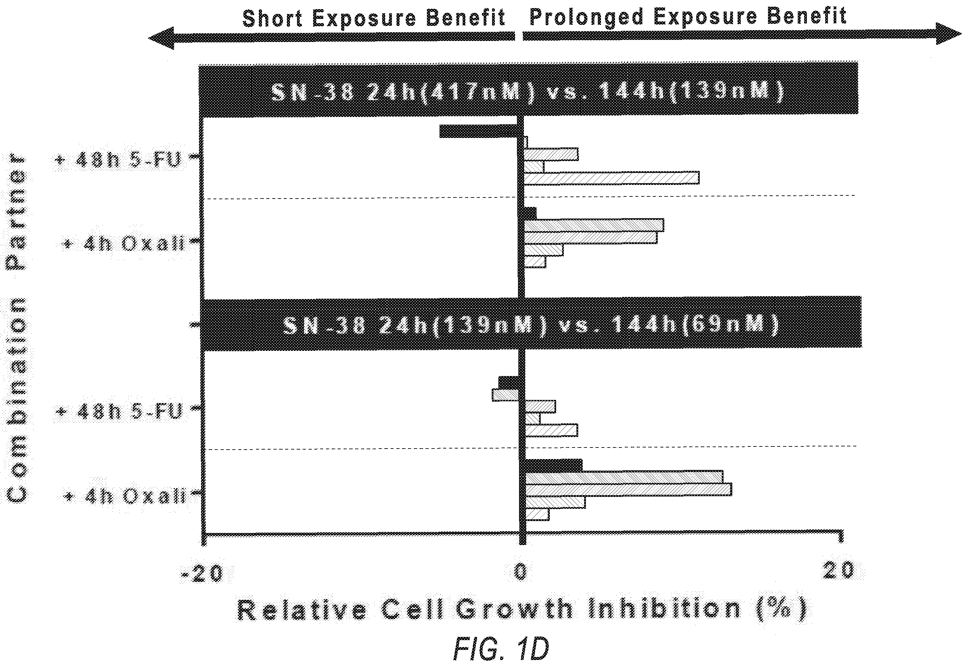

Figure 1D is a graph showing the percent relative cell growth inhibition of the cell lines tested in Figure 1C at different exposure times (4 hours or 48 hours) for different combinations of SN-38 with 5-fluorouracil (5-FU) or oxaliplatin (oxali).

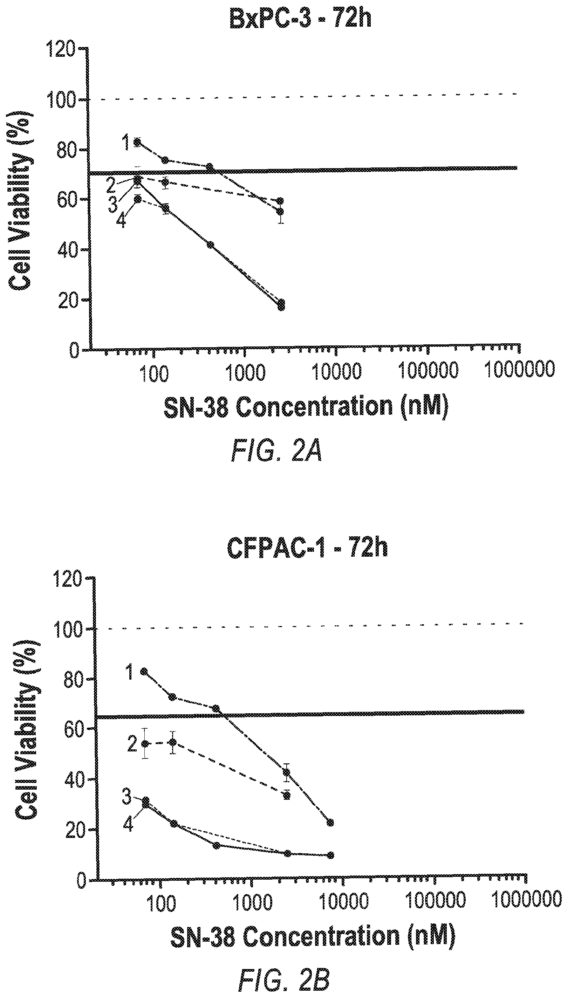

Figure 2A is a graph showing the cell viability as a function of SN-38 exposure for BxPC-3 pancreatic cancer cells.
Figure 2B is a graph showing the cell viability as a function of SN-38 exposure for CFPAC-1 pancreatic cancer cells.

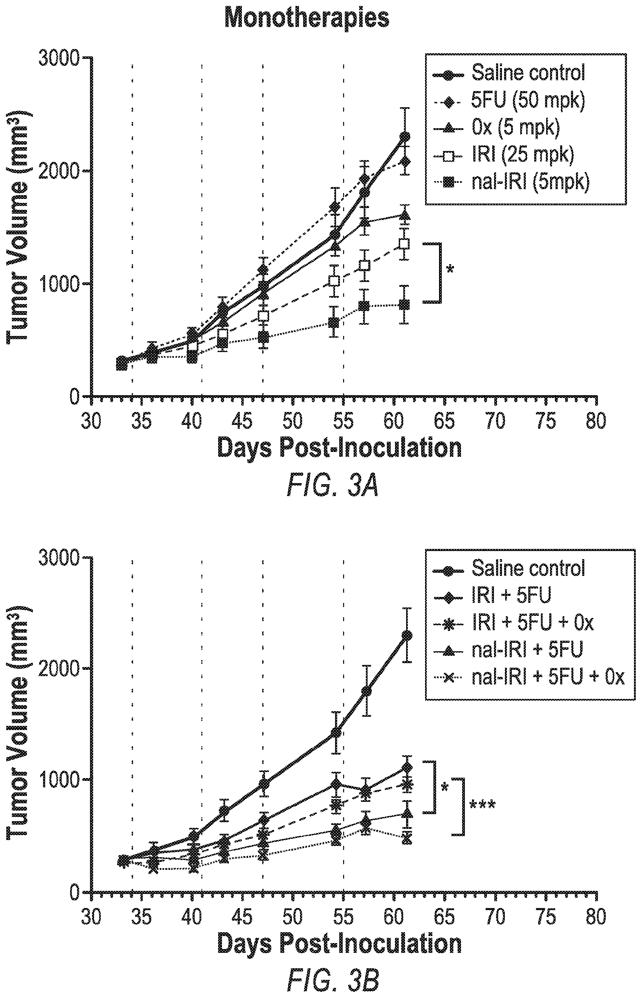

Figure 3A is a graph showing the tumor volume over time measured in a BxPC-3 pancreatic cancer xenograft mouse efficacy model after treatment with individual antineoplastic agents: including 5-fluorouracil (5FU), oxaliplatin (Ox), (non-liposomal) irinotecan (IRI) and MM-398 liposomal irinotecan (nal-IRI).
Figure 3B is a graph showing the tumor volume over time measured in a BxPC-3 pancreatic cancer xenograft mouse efficacy model after treatment with various combinations of antineoplastic agents: (non-liposomal) irinotecan (IRI) and 5FU; (non-liposomal)irinotecan (IRI), oxaliplatin and 5FU; MM-398 liposomal irinotecan (nal-IRI) and 5FU; and 398 liposomal irinotecan (nal-IRI), oxaliplatin and 5FU.

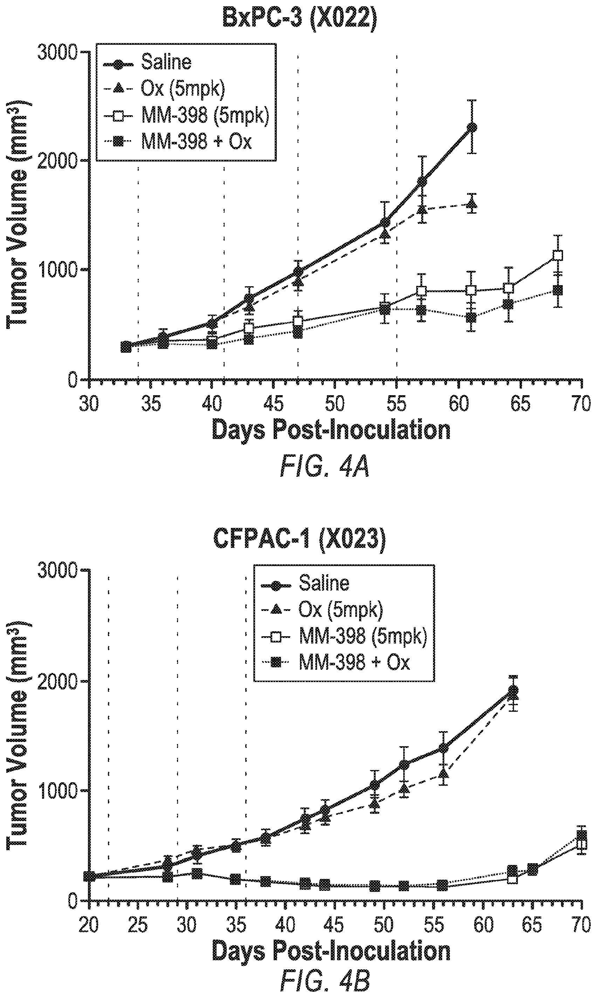

Figure 4A is a graph showing the tumor volume over time measured in a BxPC-3 pancreatic cancer xenograft mouse efficacy model after treatment with oxaliplatin monotherapy, MM-398 liposomal irinotecan (nal-IRI) monotherapy, and a combination of MM-398 liposomal irinotecan (nal-IRI) and oxaliplatin (Ox).
Figure 4B is a graph showing the tumor volume over time measured in a CFPAC-1 pancreatic cancer xenograft mouse efficacy model after treatment with oxaliplatin monotherapy, MM-398 liposomal irinotecan (nal-IRI) monotherapy, and a combination of MM-398 liposomal irinotecan (nal-IRI) and oxaliplatin (Ox).

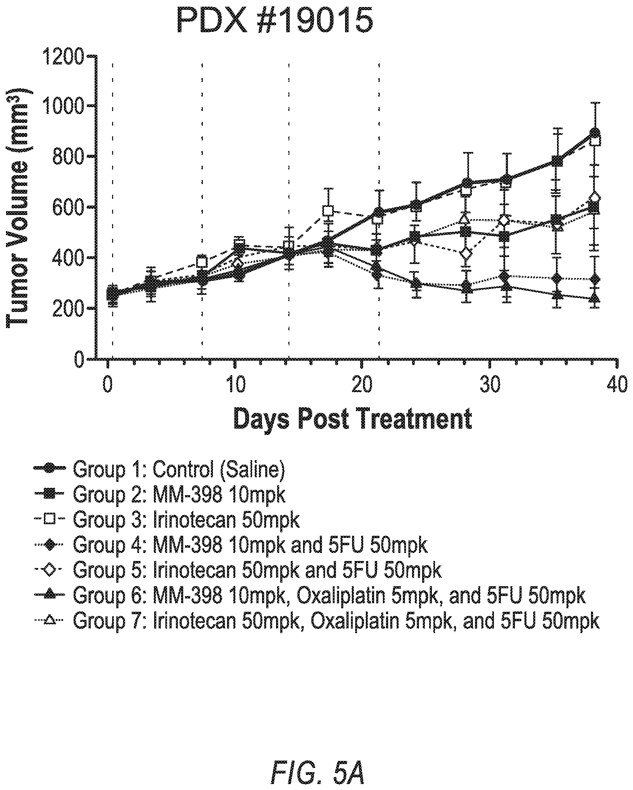

Figure 5A is a graph showing the tumor volume over time measured in a patient-derived xenograft (PDX #19015) pancreatic cancer mouse efficacy model after treatment with MM-398 liposomal irinotecan (nal-IRI) monotherapy, (non-liposomal) irinotecan monotherapy (irinotecan), and various combination therapies: MM-398 liposomal irinotecan (nal-IRI) and 5-fluorouracil (5FU); (non-liposomal) irinotecan (irinotecan) and 5FU; MM-398 liposomal irinotecan (nal-IRI), oxaliplatin and 5FU; and (non-liposomal) irinotecan, oxaliplatin and 5FU.

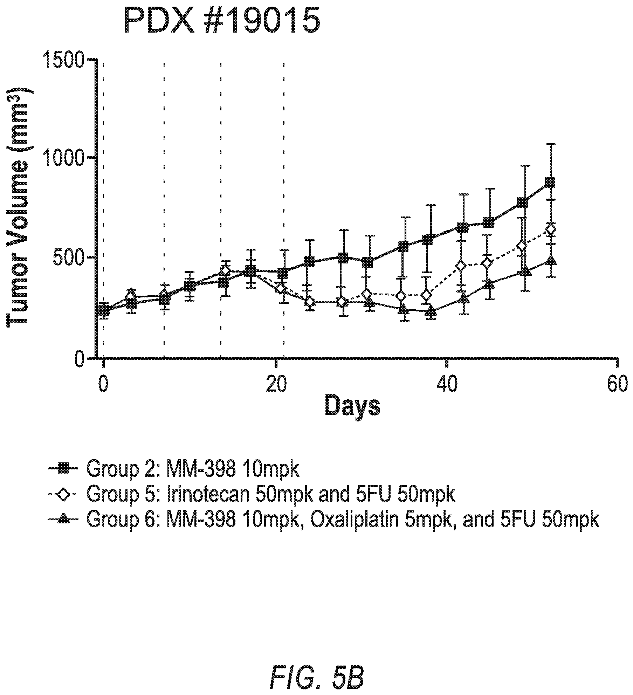

Figure 5B is a graph showing the tumor volume over time measured in a patient-derived xenograft (PDX #19015) pancreatic cancer mouse efficacy model after treatment with the MM-398 containing combination therapies shown in Figure 5A: MM-398 liposomal irinotecan (nal-IRI) and 5-fluorouracil (5FU), MM-398 liposomal irinotecan (nal-IRI), oxaliplatin and 5FU; and (non-liposomal) irinotecan, oxaliplatin and 5FU.

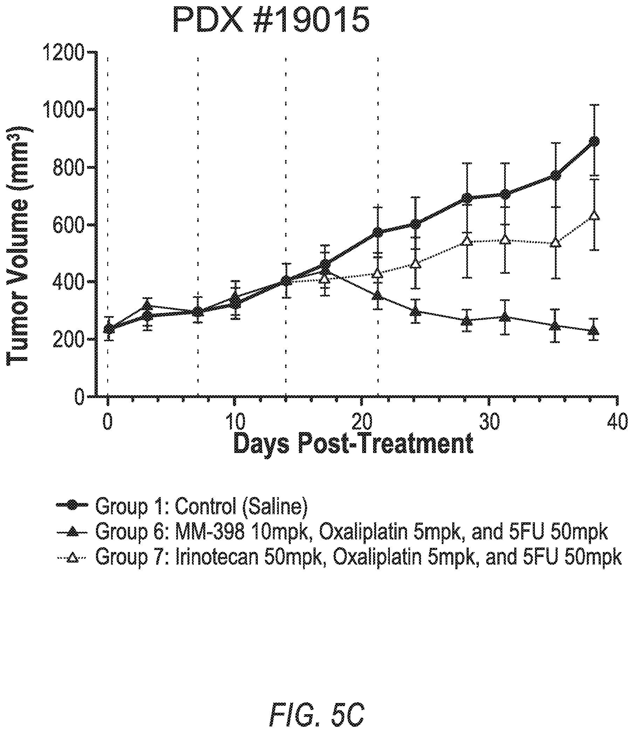

Figure 5C is a graph showing the tumor volume over time measured in a patient-derived xenograft (PDX #19015) pancreatic cancer mouse efficacy model after treatment with the oxaliplatin containing combination therapies shown in Figure 5A: MM-398 liposomal irinotecan (nal-IRI), oxaliplatin and 5FU; and (non-liposomal) irinotecan, oxaliplatin and 5FU.

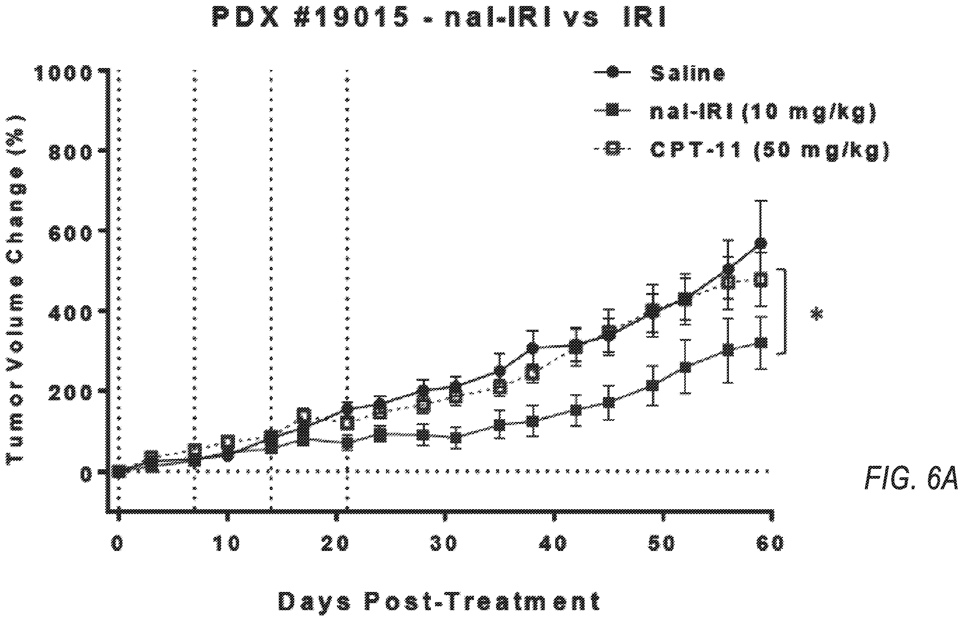

Figure 6A is a graph showing the percent tumor volume change over time measured in a patient-derived xenograft (PDX #19015) pancreatic cancer mouse efficacy model after treatment with a saline control, MM-398 liposomal irinotecan (nal-IRI) monotherapy, or (non-liposomal) irinotecan monotherapy (irinotecan).

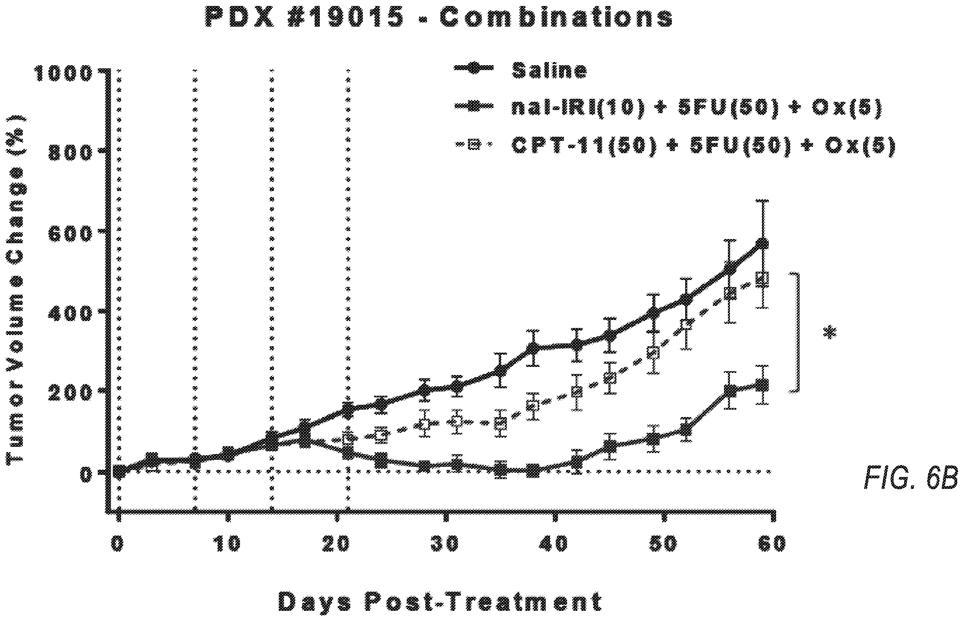

Figure 6B is a graph showing the percent tumor volume change over time measured in a patient-derived xenograft (PDX #19015) pancreatic cancer mouse efficacy model after treatment with saline control or two oxaliplatin containing combination therapies: MM-398 liposomal irinotecan (nal-IRI), oxaliplatin and 5FU; and (non-liposomal) irinotecan, oxaliplatin and 5FU.

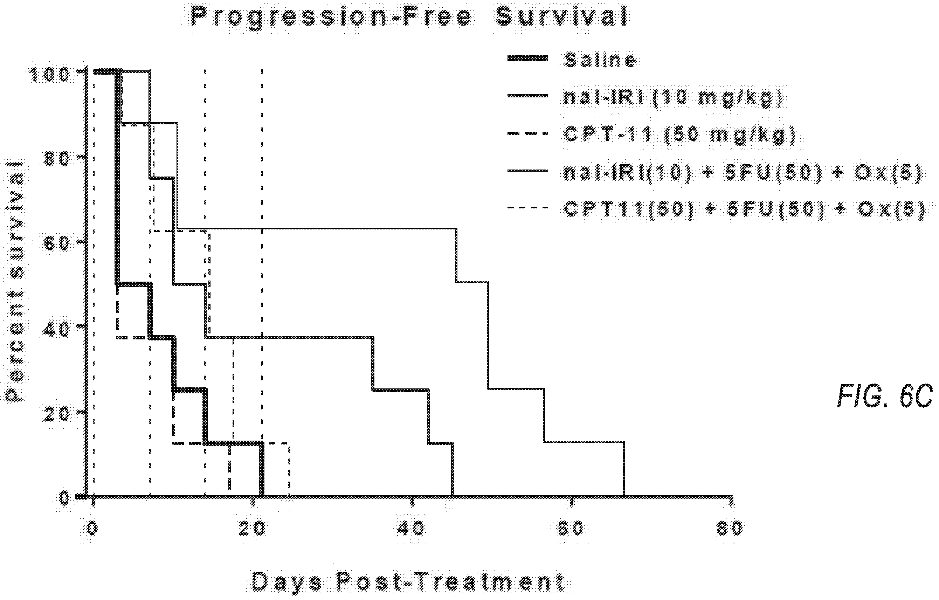

Figure 6C is a graph of the progression free survival measured in a patient-derived xenograft (PDX #19015) pancreatic cancer mouse efficacy model after treatment with two oxaliplatin containing combination therapies: MM-398 liposomal irinotecan (nal-IRI), oxaliplatin and 5FU; and (non-liposomal) irinotecan, oxaliplatin and 5FU.

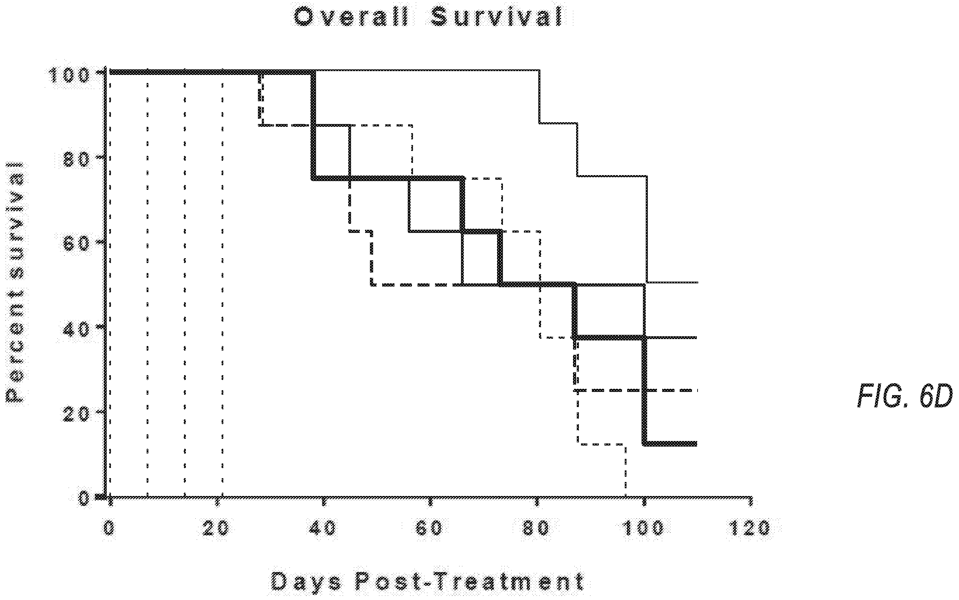

Figure 6D is a graph of the overall survival measured in a patient-derived xenograft (PDX #19015) pancreatic cancer mouse efficacy model after treatment with two oxaliplatin containing combination therapies: MM-398 liposomal irinotecan (nal-IRI), oxaliplatin and 5FU; and (non-liposomal) irinotecan, oxaliplatin and 5FU.

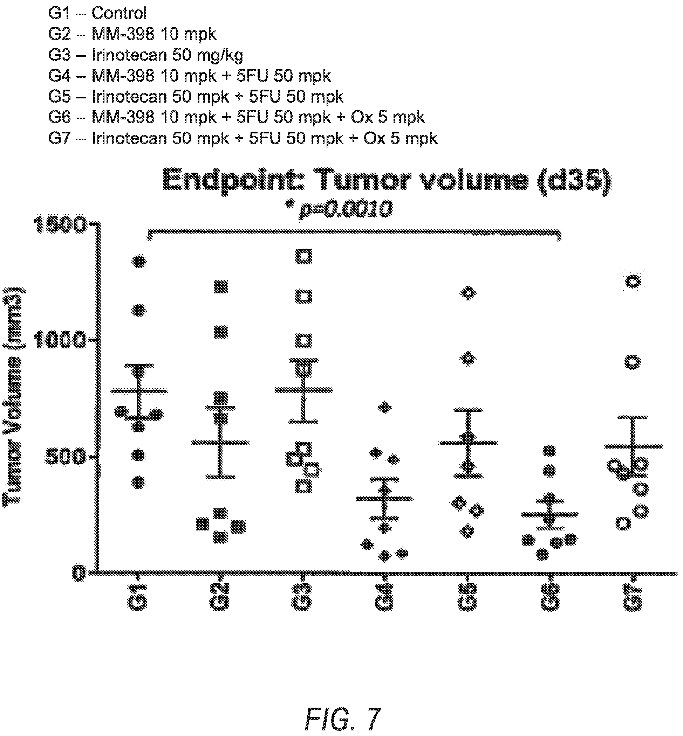

Figure 7 is a graph showing the tumor volume measured in a patient-derived xenograft (PDX #19015) pancreatic cancer mouse efficacy model after treatment with MM-398 liposomal irinotecan (nal-IRI) monotherapy, (non-liposomal) irinotecan monotherapy (irinotecan), and various combination therapies: MM-398 liposomal irinotecan (nal-IRI) and 5-fluorouracil (5FU); (non-liposomal) irinotecan (irinotecan) and 5FU; MM-398 liposomal irinotecan (nal-IRI), oxaliplatin and 5FU; and (non-liposomal) irinotecan, oxaliplatin and 5FU.

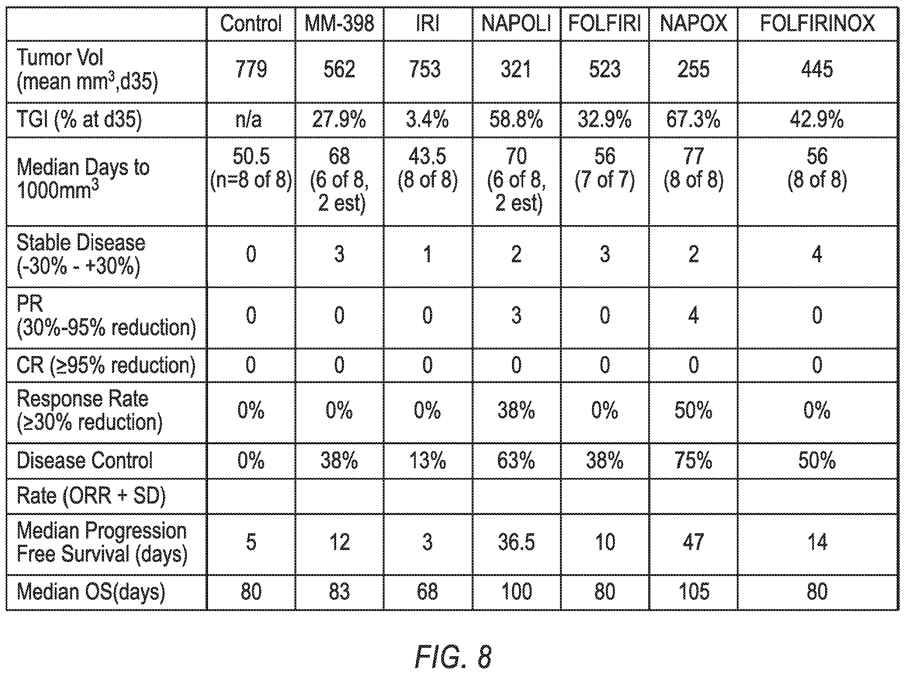

Figure 8 is a table showing the results obtained from a patient-derived xenograft (PDX #19015) pancreatic cancer mouse efficacy model after treatment with MM-398 liposomal irinotecan alone, non-liposomal irinotecan alone (monotherapy), MM-398 liposomal irinotecan in combination with 5FU (NAPOLI, double therapy), MM-398 liposomal irinotecan in combination with 5FU + oxaliplatin (NAPOX, triple therapy) and non-liposomal irinotecan combined with oxaliplatin and 5-fluorouracil (FOLFIRINOX).

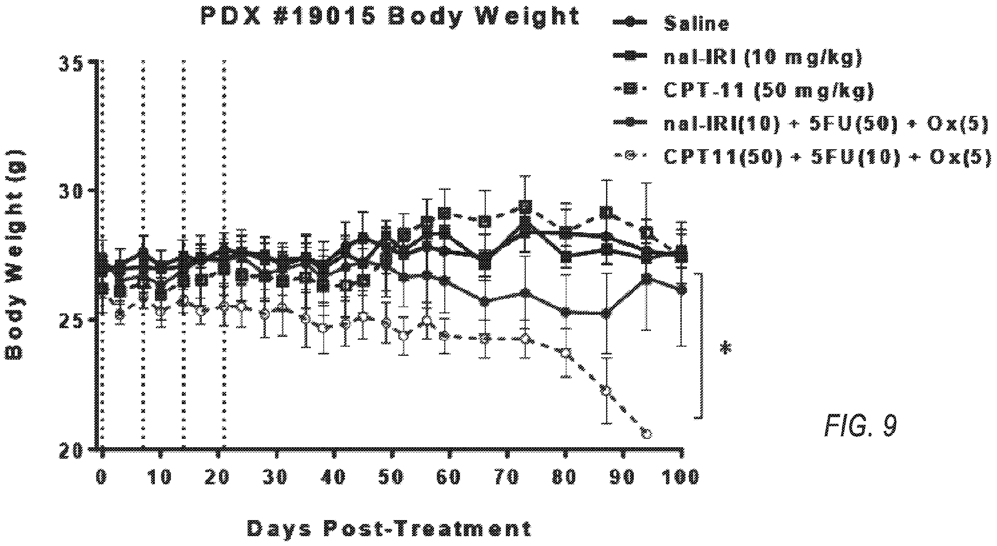

Figure 9 is a graph showing the tolerability of various therapies in a mouse model, measured by recording the body weight of the mouse after administration of a saline control, liposomal irinotecan (nal-IRI), a combination of nanoliposomal irinotecan, 5-FU and oxaliplatin or a combination of non-liposomal irinotecan (CPT11), 5FU and oxaliplatin on days 0, 7, 14 and 21.

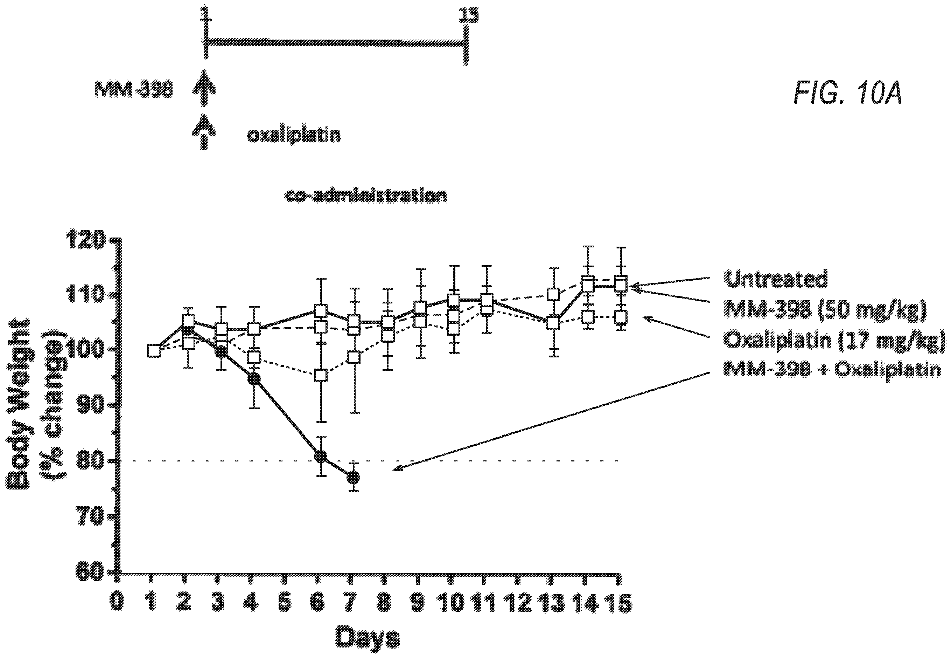

Figure 10A is a graph showing the tolerability of various therapies in a mouse model, measured by recording the body weight of the mouse after administration of high doses of MM-398 liposomal irinotecan (nal-IRI), oxaliplatin and a combination of MM-398 liposomal irinotecan and oxaliplatin given together on the same day.

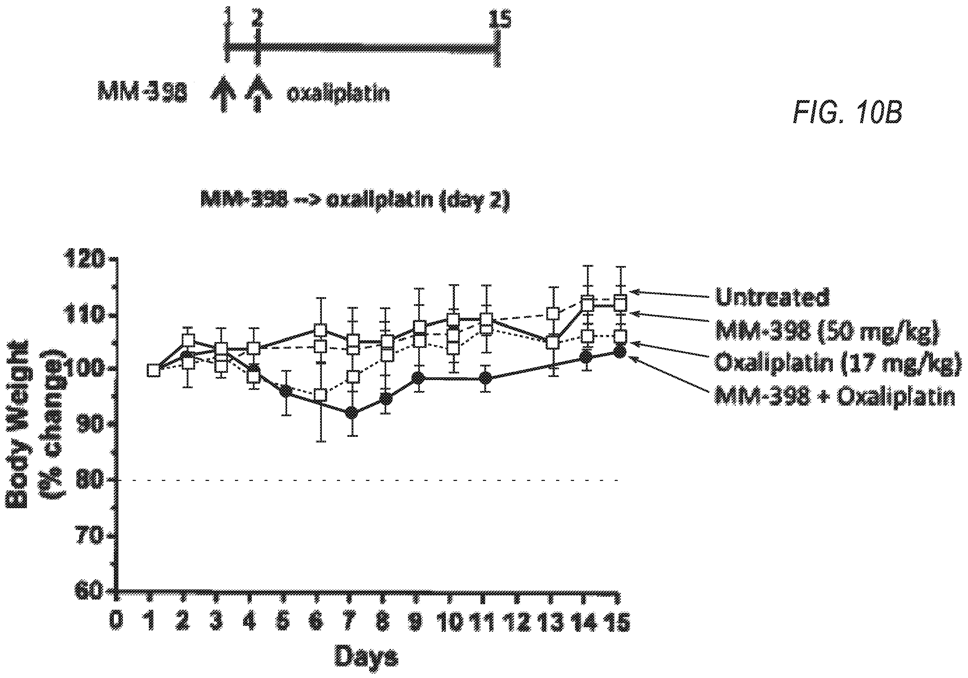

Figure 10B is a graph showing the tolerability of various therapies in a mouse model, measured by recording the body weight of the mouse after administration of high doses of MM-398 liposomal irinotecan (nal-IRI), oxaliplatin and a combination of MM-398 liposomal irinotecan and oxaliplatin given sequentially on separate successive days with the MM-398 administered on day 1 and the oxaliplatin administered on day 2.

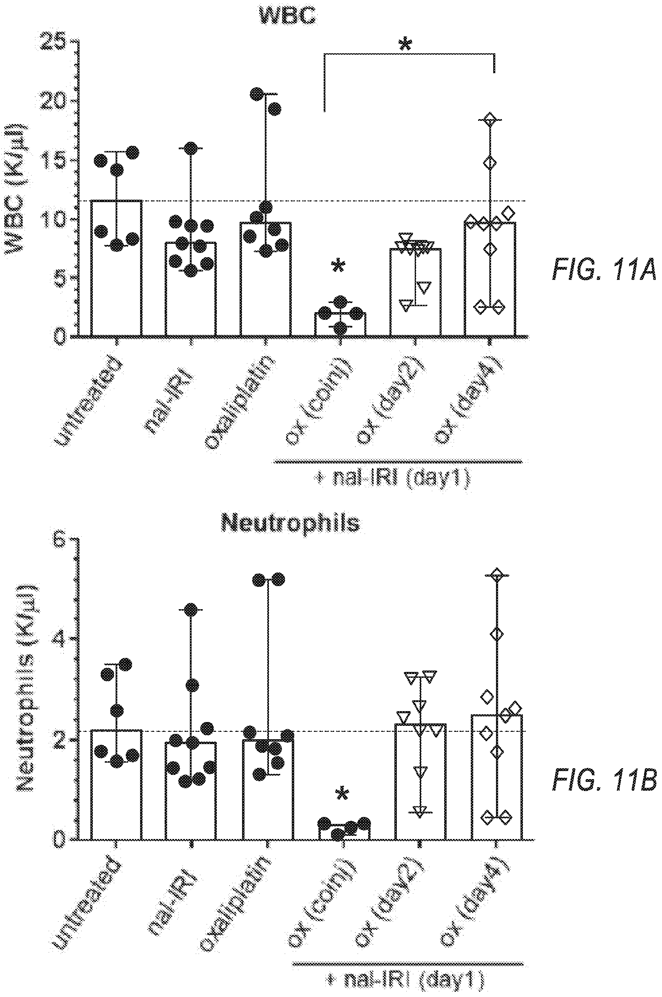

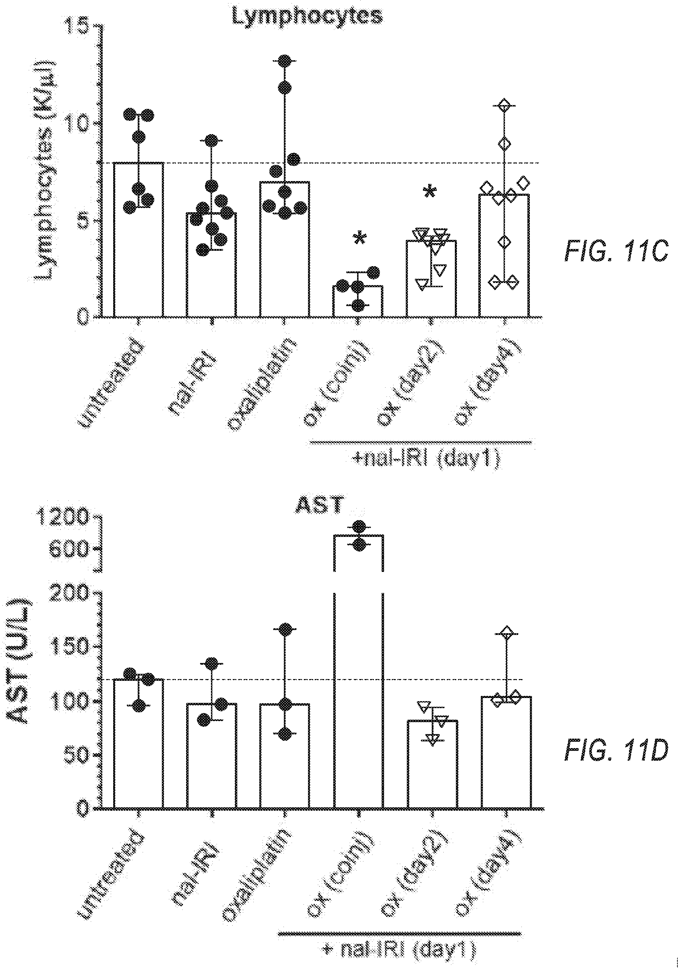

Figures 11A, 11B and 11C are bar graphs depicting hematological toxicities observed in mice after administration of high doses of MM-398 liposomal irinotecan (nal-IRI) and oxaliplatin administered on the same day or with oxaliplatin administered at least one day after administration of MM-398: A. White blood cells; B. Neutrophils; and C. Lymphocytes.

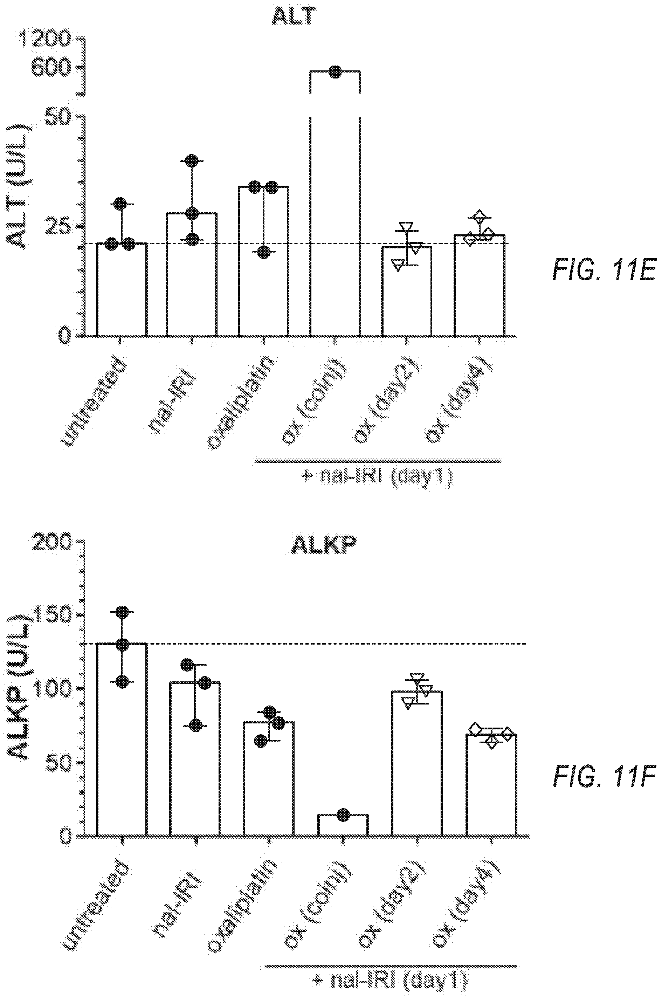

Figures 11D, 11E and 11F is bar graphs depicting liver enzyme levels observed in mice after administration of high doses of MM-398 liposomal irinotecan (nal-IRI) and oxaliplatin administered on the same day or with oxaliplatin administered at least one day after administration of MM-398: D. aspartate aminotransferase (AST); E. alanine transaminase (ALT); F. alkaline phosphatase (ALKP).

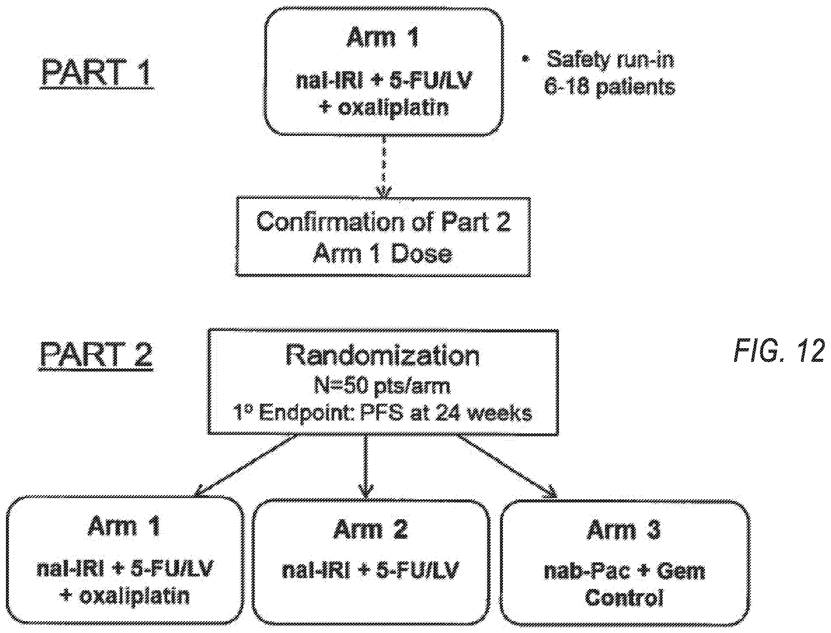

Figure 12 is a schematic of methods of treating pancreatic cancer, including methods comprising the administration of liposomal irinotecan, oxalipaltin, 5-fluorouracil and leucovorin.

### DETAILED DESCRIPTION

Unless otherwise indicated, the dose of liposomal irinotecan or irinotecan liposome as recited herein refers to the amount of irinotecan hydrochloride trihydrate providing an amount of irinotecan encapsulated in the liposome of the liposomal irinotecan or irinotecan liposome. For example, a dose of 60 mg/m² liposomal irinotecan refers to an amount of the liposomal irinotecan providing the same amount of liposome encapsulated irinotecan that is present in 60 mg/m² of irinotecan hydrochloride trihydrate, and is equivalent to a dose of about 50 mg/m² of liposomal irinotecan based on the amount of the irinotecan free base encapsulated in the liposomal irinotecan.

As used herein, unless otherwise indicated, the term "nal-IRI" (nanoliposomal irinotecan) and "MM-398" refer to a form of liposomal irinotecan. The term "CPT-11" refers to (non-liposomal) irinotecan hydrochloride trihydrate.

As used herein, "5-FU" and "5FU" and used interchangeably and refer to 5-fluorouracil.

Using pancreatic cancer cell lines (Example 1), we demonstrated enhanced cell death when liposomal irinotecan treatment is simulated using prolonged exposure of SN-38 (the active metabolite of irinotecan) in combination with 5-FU and oxaliplatin. Figure 1 shows that prolonged exposure of SN-38 simulates MM-398 treatment in vitro. Referring to Figure 1A, MM-398 treatment results in prolonged tumor exposure to the active metabolite, SN-38, compared to non-liposomal irinotecan (CPT-11). Referring to Figure 1B, prolonged low-dose exposure of SN-38 mimics MM-398 tumor delivery in vitro. Referring to Figure 1C, prolonged low-dose exposure resulted in greater cell growth inhibition in multiple pancreatic cancer cell lines. The graph comprises four sections, and for each section the cell line data is presented with AsPC-1 data at the top, followed next by BxPC-3, Capan-2, CFPAC-1, and finally MaPaCa-2 on the bottom. Referring to Figure 1D, the benefit of prolonged exposure to low concentrations of SN-38 was also observed when combined with 5-FU (20.7 mM for 48h) or oxaliplatin (12.3 mM for 4h). Both combinations also increased sensitivity of resistance cell lines to prolonged low-dose SN-38.

Figure 2 is two line graphs that depict cell viability following treatment with SN-38 as a single agent or the combination of SN-38 and oxaliplatin. BxPC-3 (Figure 2A) or CFPAC-1 (Figure 2B) cells were treated for 4h or 72h, washed and then incubated for an additional 24h or 144h with fresh media, following which cell viability was assessed. The data traces are labeled "1" (SN-38 alone for four hours followed by a 24 hour incubation; "2" SN-38 + oxaliplatin for four hours followed by a 24 hour incubation; "3" SN-38 alone for 72 hours followed by a 144 hour incubation; and "4" SN-38 + oxaliplatin for 72 hours followed by a 144 hour incubation. Treatment of the cells with a combination of SN-38 and oxaliplatin decreased the IC-50 when cells were treated for 4h only as compared to treatment with single agents in both cell lines tested.

Testing of cell line-derived and patient-derived xenograft models of pancreatic cancer in Example 2 demonstrated improved anti-tumor activity of liposomal irinotecan relative to exposure-matched doses of non-liposomal irinotecan. In the mouse animal studies in Example 2, a dose of "x" mg/kg liposomal irinotecan provides about the same exposure to the topoisomerase 1 inhibitor (irinotecan and/or SN-38) as a dose of "5x" non-liposomal irinotecan (CPT-11). The liposomal irinotecan consistently improved tumor growth inhibition and survival relative to non-liposomal irinotecan in preclinical models, both as a monotherapy and in combination with 5-FU and oxaliplatin. The addition of MM-398 to 5-FU and/or oxaliplatin did not exacerbate the baseline toxicities of these agents, including weight loss and neutropenia, and tolerability could be further improved by delaying the administration of oxaliplatin to 1 day post-MM-398. These findings illustrate the therapeutic potential of liposomal irinotecan in combination with 5-FU/LV and oxaliplatin and support an ongoing Phase 2 trial (NCT02551991) of this triplet regimen in first-line PDAC (Example 2).

An animal model of the FOLFIRINOX regimen was tested against the MM-398 + 5-FU/LV + oxaliplatin regimen in a pancreatic tumor xenograft mouse model. Liposomal irinotecan (MM-398) performed better than conventional (non-liposomal) irinotecan (CPT-11) at equivalent exposure doses (5 mg/kg MM-398 vs. 25 mg/kg free IRI) in the BxPC-3 pancreatic xenograft cancer models (Example 2) either alone (e.g., Figure 3A), or in combination with oxaliplatin and/or 5-FU (e.g., Figure 3B).

In the mouse model tested in Example 2, efficacy of MM-398 in a 5-FU insensitive pancreatic cancer model (BxPC-3) was evaluated. Cancer cells were implanted subcutaneously in mice; when tumors were well established and had reached mean volumes of ~300 mm³, IV treatment with free irinotecan (IRI), MM-398, 5-FU, oxaliplatin (Ox) or control was initiated. Doses are indicated above for each treatment, and were given weekly x4 weeks, at time points indicated by dashed lines on graphs. Figure 3A depicts a line graph representing tumor growth after treatment with various individual treatment agents. Figure 3B depicts a line graph representing tumor growth after treatment with various combinations of treatment agents.

Efficacy of MM-398 in a 5-FU insensitive pancreatic cancer model (BxPC-3). Cancer cells were implanted subcutaneously in mice; when tumors were well established and had reached mean volumes of ~300 mm³, IV treatment with doublet or triplet regimens containing either IRI or MM-398 in combination with oxaliplatin and/or 5-FU was initiated. Doses are indicated above for each treatment, and were given weekly x4 weeks, at time points indicated by dashed lines on graphs. In comparison to Figure 4A (discussed below), doublet or triplet regimens containing either IRI or MM-398 in combination with oxaliplatin and/or 5-FU demonstrate that the MM-398-containing doublet and triplet regimens inhibit tumor growth significantly better than the IRI-containing regimens. The addition of oxaliplatin to the doublet combinations of FOLFIRI or MM-398+5-FU/LV causes a slight increase in tumor growth inhibition (Figure 3B: compare IRI + 5FU to IRI + 5FU +Ox for FOLFIRI vs. FOLFIRINOX; compare nal-IRI + 5FU to nal-IRI + 5FU + Ox for MM-398+5-FU/LV vs. MM-398+5-FU/LV+Ox). However, comparison of FOLFIRI versus the MM-398+5-FU/LV doublet (IRI + 5FU vs. nal-IRI + 5FU), and FOLFIRINOX vs. the MM-398+5-FU/LV+Ox triplet (IRI + 5FU +Ox vs. nal-IRI + 5FU + Ox ), demonstrates significantly more tumor growth inhibition with the MM-398-containing regimens. Further, the MM-398-containing doublet regimen performed better than the FOLFIRINOX triplet (nal-IRI + 5FU vs. IRI + 5FU +Ox), owing to the improved efficacy of MM-398 compared to conventional irinotecan.

Single agent results of the individual treatments are shown in Figure 4A, demonstrating that MM-398 significantly inhibits tumor growth compared to free IRI. Figures 4A and 4B are two line graphs depicting tumor growth in mouse xenograft models following intravenous treatment with saline (control, circles), 5 mg/kg oxaliplatin (triangles), 5 mg/kg MM-398 (light squares), or the combination of BxPC-3 (Figure 4A) or CFPAC-1 (Figure 4B) tumor cells were implanted subcutaneously in mice. Treatment was initiated after tumors were well established, and treatments were given four times (BxPC-3 model) or three times (CFPAC-1 model) at the time points indicated by dashed lines on the graphs.

Figures 5A, 5B, 5C, 6A, 6B, 6C, 6D and 7 are graphs obtained by measuring tumor growth inhibition in mice following various treatments. Tumor cells (PDX model 19015) were implanted subcutaneously in mice. When tumors were well-established, and had reached a mean volume of ~250 mm³, IV treatment with MM-398 or non-liposomal irinotecan alone, or in combination with 5-FU or 5-FU + oxaliplatin, was initiated. Treatment doses are indicated in the figure beside each treatment, and were given 4 times.

Figures 5A-5C are three line graphs depicting tumor growth inhibition in mice following various treatments. Tumor cells, PDX 19015 model, were implanted subcutaneously in mice. When tumors were well-established, and had reached a mean volume of ~250 mm³, IV treatment with MM-398 or non-liposomal irinotecan as monotherapy, or in combination with 5-FU and Oxaliplatin, was initiated. Treatment doses are indicated in the legend beside each treatment, and were given four times, at time points indicated by dashed lines on the graphs. The addition of 5-FU to MM-398 or non-liposomal irinotecan significantly improved tumor growth inhibition relative to the respective monotherapies. The addition of oxaliplatin to MM-398 + 5-FU further improves response by significantly delaying tumor progression as compared to MM-398 monotherapy. The delay in tumor progression was not significant in the group treated with the double therapy of MM-398 + 5-FU. Figure 5A is a line graph comprising data from all of the combinations (both those with MM-398 and those with irinotecan), and shows that the combination of MM-398, oxaliplatin, and 5-FU resulted in the most inhibition of tumor growth (lowest line trace), although the combination of MM-398 and 5-FU also inhibited tumor growth (next lowest line). Figure 5B is a line graph comprising data from the MM-398 combinations only (no irinotecan combinations or control line) for the purpose of comparison. As can be seen in the graph, the triple combination treatment resulted in the most tumor growth inhibition (lowest line), and the double combination of irinotecan and 5-FU (middle line) was better than MM-398 alone (highest line) in inhibiting tumor growth. Figure 5C is a subset of the same data that allows comparison of the oxaliplatin combinations to the saline control.

Figure 6A is a graph showing the percent tumor volume change over time measured in a PDX 19015 pancreatic cancer xenograft mouse efficacy model after treatment with a saline control, MM-398 liposomal irinotecan (MM-398) monotherapy, or (non-liposomal) irinotecan monotherapy (irinotecan). The data in Figure 6A shows a significantly greater reduction in the percent tumor volume change for administration of 10 mg/kg liposomal irinotecan (MM-398) compared to non-liposomal irinotecan (CPT-11) at 50 mg/kg, each administered on days 0, 7, 14 and 21 followed by observation for a total of about 60 days. Figure 6B is a graph showing the percent tumor volume change over time measured in a PDX 19015 pancreatic cancer xenograft mouse efficacy model after treatment with saline control or two oxaliplatin containing combination therapies: MM-398 liposomal irinotecan (MM-398), oxaliplatin and 5FU; and (non-liposomal) irinotecan, oxaliplatin and 5FU. Mice receiving the combination of liposomal irinotecan (MM-398, also called MM-398) with 5FU and oxaliplatin on days 0, 7, 14 and 21 showed significantly reduced tumor volume percent change through the observation period of about 60 days, compared to mice receiving the combination of non-liposomal irinotecan (CPT-11) with oxaliplatin and 5-FU on days 0, 7, 14 and 21. Referring to Figure 6C, the addition of oxaliplatin to MM-398 + 5-FU significantly improves progression free survival of mice bearing PDX 19015 tumors, as compared to the control group and MM-398 monotherapy. The difference between MM-398 + 5FU and MM-398 monotherapy is not statistically significant. Referring to Figure 6D, the addition of 5-FU and oxaliplatin to MM-398 significantly improve overall survival relative to the control group. No benefit of added 5-FU or oxaliplatin was observed with non-liposomal irinotecan. Referring to Figure 7, the addition of oxaliplatin to MM-398 + 5-FU significantly delays tumor progression relative to MM-398 monotherapy, as indicated by significantly reduced tumor volume at day 35.

Figure 8 is a table showing results of tumor growth and survival in mice following various treatments. Tumor cells (PDX 19015 model) were implanted subcutaneously in mice. When tumors were well-established, and had reached a mean volume of ~250 mm³, IV treatment with MM-398 or non-liposomal irinotecan alone (monotherapy), or in combination with 5-FU (NAPOLI, double therapy) or 5-FU + oxaliplatin (NAPOX, triple therapy), was initiated.

Mice treated with the triple therapy, NAPOX (50%) had the best Overall Response Rate (ORR), as compared to double NAPOLI (38%), or monotherapy MM-398 monotherapy (0%). Further, triple therapy treated mice also had a better Disease Control Rate (DCR): NAPOX (75%), NAPOLI (63%), MM-398 monotherapy (38%), and Progression Free Survival (PFS): NAPOX was 47 days, relative to 36.5 days for NAPOLI and 12 days for MM-398 monotherapy. NAPOX PFS was significantly better than the monotherapy, whereas NAPOLI is not significantly better than the monotherapy. Notably, the combination of liposomal irinotecan with 5FU and oxaliplatin was better tolerated than the combination of an SN-38 exposure-matched dose of non-liposomal irinotecan with 5FU and oxaliplatin in a mouse tolerability study over 100 days. Figure 9 is a graph showing the body weight of mice after administration of various regimens: a saline control, liposomal irinotecan (MM-398), a combination of nanoliposomal irinotecan, 5-FU and oxaliplatin or a combination of non-liposomal irinotecan (CPT11), 5FU and oxaliplatin. Liposomal irinotecan improved tolerability in a mouse model following repeated dosing in mice relative to non-liposomal irinotecan when combined with 5-FU and oxaliplatin. Significance was determined by ordinary 2-way analysis of variance (ANOVA). The regimens were administered on days 0, 7, 14 and 21 of the study. The administration of 10 mg/kg liposomal irinotecan and the 50 mg/kg dose of non-liposomal free irinotecan (CPT11) provide a comparable dose of SN-38 to tumor cells in the mouse model.

The tolerability of combinations of MM-398 liposomal irinotecan and oxaliplatin was improved in mouse models when the oxaliplatin was administered one day after the administration of the MM-398. Figures 10A and 10B depict line graphs demonstrating the toxicities associated with MM-398 and oxaliplatin given as monotherapy or combined therapy given concurrently (A) or staggered, with oxaliplatin given 1 day after MM-398 administration (B). Co-administration of MM-398 and oxaliplatin leads to significant toxicities as measured by loss of body weight, whereas delaying oxaliplatin administration by 24h after MM-398 does not lead to significant changes in body weight.

Figure 11A-11F are bar graphs depicting hematological and liver toxicities following treatment with MM-398 with or without oxaliplatin given either concurrently or sequentially with MM-398. Hematological toxicities (A-C) were improved by delayed administration of oxaliplatin. Liver enzymes (D-F) remained comparable to monotherapies when oxaliplatin administration was delayed.

These preclinical findings support the therapeutic use of liposomal irinotecan in combination with 5-FU/LV and oxaliplatin and an ongoing Phase 2 trial (NCT02551991) of this triplet regimen in first-line PDAC (Example 2). Figure 12 depicts a graphical representation of the study design employing the combination of MM-398 + 5-FU/LV + oxaliplatin in (Arm 1) and MM-398 + 5-FU/LV (Arm 2), and nab-paclitaxel + gemcitabine (Arm 3) as described herein.

For example, use of a combination of liposomal irinotecan, oxaliplatin, and 5-fluorouracil in treating metastatic adenocarcinoma of the pancreas in a human patient who has not previously received chemotherapy to treat the metastatic adenocarcinoma of the pancreas, the use comprising administering an antineoplastic therapy to the patient a total of once every two weeks, the antineoplastic therapy consisting of: (a) 60 mg/m² of liposomal irinotecan, 60 mg/m² oxaliplatin, 200 mg/m² of (l)-form of leucovorin or 400 mg/m² of the (l+d) racemic form of leucovorin, and 2,400 mg/m² 5-fluorouracil to treat the metastatic adenocarcinoma of the pancreas in the human patient; (b) 60 mg/m² of liposomal irinotecan, 85 mg/m² oxaliplatin, 200 mg/m² of (I)-form of leucovorin or 400 mg/m² of the (I+d) racemic form of leucovorin, and 2,400 mg/m² 5-fluorouracil to treat the metastatic adenocarcinoma of the pancreas in the human patient; (c) 60 mg/m² of liposomal irinotecan, 60 mg/m² oxaliplatin, 200 mg/m² of (I)-form of leucovorin or 400 mg/m² of the (l+d) racemic form of leucovorin, and 2,400 mg/m² 5-fluorouracil to treat the metastatic adenocarcinoma of the pancreas in the human patient wherein the liposomal irinotecan, oxaliplatin and leucovorin is administered on days 1 and 15 of a 28-day treatment cycle; (d) 60 mg/m² of liposomal irinotecan, 85 mg/m² oxaliplatin, 200 mg/m² of (l)-form of leucovorin or 400 mg/m² of the (I+d) racemic form of leucovorin, and 2,400 mg/m² 5-fluorouracil to treat the metastatic adenocarcinoma of the pancreas in the human patient, wherein the liposomal irinotecan, oxaliplatin and leucovorin is administered on days 1 and 15 of a 28-day treatment cycle; (e) 60 mg/m² of liposomal irinotecan, 60 mg/m² oxaliplatin, 200 mg/m² of (I)-form of leucovorin or 400 mg/m² of the (I+d) racemic form of leucovorin, and 2,400 mg/m² 5-fluorouracil to treat the metastatic adenocarcinoma of the pancreas in the human patient wherein the liposomal irinotecan is administered, followed by administering the oxaliplatin, followed by administering the leucovorin, followed by administering the 5-fluorouracil; (f) 60 mg/m² of liposomal irinotecan, 85 mg/m² oxaliplatin, 200 mg/m² of (l)-form of leucovorin or 400 mg/m² of the (I+d) racemic form of leucovorin, and 2,400 mg/m² 5-fluorouracil to treat the metastatic adenocarcinoma of the pancreas in the human patient wherein the liposomal irinotecan is administered, followed by administering the oxaliplatin, followed by administering the leucovorin, followed by administering the 5-fluorouracil; or (g) 60 mg/m² of liposomal irinotecan, 60 mg/m²-85mg/m² oxaliplatin, 200 mg/m² of (I)-form of leucovorin or 400 mg/m² of the (I+d) racemic form of leucovorin, and 2,400 mg/m² 5-fluorouracil to treat the metastatic adenocarcinoma of the pancreas in the human patient wherein the liposomal irinotecan, oxaliplatin and leucovorin is administered on days 1 and 15 of a 28-day treatment cycle, wherein the liposomal irinotecan is administered, followed by administering the oxaliplatin, followed by administering the leucovorin, followed by administering the 5-fluorouracil, wherein the administration of the oxaliplatin begins 2 hours after completing each administration of the liposomal irinotecan. Each of these exemplary uses can be modified to replace the doses of liposomal irinotecan, oxaliplatin, leucovorin and 5-flurouracil disclosed herein in the following passages relating to these specific components. Sometimes the liposomal irinotecan comprises irinotecan sucrose octasulfate encapsulated in liposomes. Sometimes, the liposomal irinotecan comprises irinotecan encapsulated in liposome vesicles consisting of 1,2-distearoyl-sn-glycero-3-phosphocholine (DSPC), cholesterol, and a N-(carbonylmethoxypolyethlyene glycol-2000)-1,2-distearoly-sn-glycero-3-phosphoethanolamine (MPEG-2000-DSPE).

Irinotecan is administered in an irinotecan liposome preparation , which is "irinotecan sucrose octasulfate salt liposome injection" . The formulation referred to herein as "MM-398" (also known as PEP02, see US 8,147,867) is a form of "nanoliposomal irinotecan" (also called "irinotecan liposome" or "liposomal Irinotecan"). MM-398 is irinotecan as the irinotecan sucrose octasulfate salt encapsulated in a nanoliposome drug delivery system.

The liposomal irinotecan can be a pharmaceutical composition prepared for human intravenous administration. For example, the liposomal irinotecan may be provided as a sterile, injectable parenteral liquid for intravenous injection. The required amount of liposomal irinotecan may be diluted, e.g., in 500 mL of 5% dextrose injection USP, to provide a variety of concentrations, for example, 5 mg/mL, and may be infused over a 90 minute period.

The active ingredient of the MM-398 injection, irinotecan, is a member of the topoisomerase I inhibitor class of drugs and is a semi-synthetic and water soluble analog of the naturally-occurring alkaloid, camptothecin. Topoisomerase I inhibitors work to arrest uncontrolled cell growth by preventing the unwinding of DNA and therefore preventing replication. The pharmacology of irinotecan is complex, with extensive metabolic conversions involved in the activation, inactivation, and elimination of the drug. Irinotecan is a pro-drug that is converted by nonspecific carboxylesterases into a 100-1000 fold more active metabolite, SN-38. SN-38 is cleared via glucuronidation, (for which major pharmacogenetic differences have been shown), and biliary excretion. These drug properties contribute to the marked differences in efficacy and toxicity observed in clinical studies with irinotecan.

The liposomal irinotecan can be a unilamellar lipid bilayer vesicle of approximately 80-140 nm in diameter that encapsulates an aqueous space that contains irinotecan complexed in a gelated or precipitated state as a salt with sucrose octasulfate. The lipid membrane of the liposome is composed of phosphatidylcholine, cholesterol, and a polyethyleneglycol-derivatized phosphatidyl-ethanolamine in the amount of approximately one polyethyleneglycol (PEG) molecule for every 200 phospholipid molecules.

The amount of liposomal irinotecan administered to the human patient can range from about 40 mg/m² to about 180 mg/m². In the invention, 60 mg/m² is administered in combination with oxaliplatin and 5-fluorouracil for treatment of pancreatic cancer (dose expressed in terms of the amount of irinotecan hydrochloride trihydrate salt). The plasma pharmacokinetics of total irinotecan and total SN-38 were evaluated in patients with cancer who received MM-398, as a single agent or as part of combination chemotherapy, at doses between 50 and 155 mg/m² (amount of irinotecan base, equivalent to 60 -180 mg/m² dose expressed in terms of the amount of irinotecan hydrochloride trihydrate salt) and 353 patients with cancer using population pharmacokinetic analysis. Over the dose range of 50 to 155 mg/m², the Cₘₐₓ and AUC of total irinotecan increases with dose. Additionally, the Cₘₐₓ of total SN-38 increases proportionally with dose; however, the AUC of total SN-38 increases less than proportionally with dose.

The combination treatment described herein encompasses administration of MM-398 liposomal irinotecan in combination with multiple additional active agents: oxaliplatin, leucovorin and 5-fluorouracil, in doses and schedules to human patients with metastatic pancreatic cancer not previously treated with a prior chemotherapeutic agent in the metastatic setting as described herein.

5-Fluorouracil is a pyrimidine antagonist that interferes with nucleic acid biosynthesis. The deoxyribonucleotide of the drug inhibits thymidylate synthetase, thus inhibiting the formation of thymidylic acid from deoxyuridylic acid, thus interfering in the synthesis of DNA. It also interferes with RNA synthesis. An exemplary effective amount of 5-fluorouracil administered to a human patient can range from about 2,000 mg/m² to about 3,000 mg/m². In the invention, the amount of 5-fluorouracil administered to the human patient is 2,400 mg/m².

Leucovorin is optionally administered prior to the 5-fluorouracil. Leucovorin acts as a biochemical cofactor for 1-carbon transfer reactions in the synthesis of purines and pyrimidines. Leucovorin does not require the enzyme dihydrofolate reductase (DHFR) for conversion to tetrahydrofolic acid. The effects of methotrexate and other DHFR-antagonists are inhibited by leucovorin. Leucovorin can potentiate the cytotoxic effects of fluorinated pyrimidines (i.e., fluorouracil and floxuridine). After 5-FU is activated within the cell, it is accompanied by a folate cofactor, and inhibits the enzyme thymidylate synthetase, thus inhibiting pyrimidine synthesis. Leucovorin increases the folate pool, thereby increasing the binding of folate cofactor and active 5-FU with thymidylate synthetase. Leucovorin has dextro- and levo-isomers, only the latter one being pharmacologically useful. As such, the bioactive levo-isomer ("levo-leucovorin") has also been approved by the FDA for treatment of cancer. The dosage of leucovorin is that of the racemic mixture containing both dextro (d) and levo (l) isomers, or optionally the (l) form of leucovorin at half the dosage of the (I + d) racemic form. An exemplary effective amount of leucovorin administered to the human patient can include an amount of (I)-form leucovorin ranging from about 100 mg/m² to about 300 mg/m². In some embodiments, the amount of (I)-form leucovorin administered to the human patient is 200 mg/m². In other embodiments, the leucovorin administered is the (I + d)-form of leucovorin, in an amount of 400 mg/m². In the invention, 200 mg/m² of the (l)-form of leucovorin or 400 mg/m² of the (I+d) racemic form of leucovorin is administered to the human patient.

Oxaliplatin is a platinum-based drug that acts as a DNA cross-linking agent to effectively inhibit DNA replication and transcription, resulting in cytotoxicity which is cell-cycle nonspecific. Oxaliplatin is typically used in combination with infusional 5-FU/LV, and is approved for use in advanced colorectal cancer (refer to package insert for more details). The effective amount of oxaliplatin administered to the human patient can range from about 30 mg/m² to about 150 mg/m², for example, from about 40 mg/m² to about 100 mg/m², or an amount of oxaliplatin of 50 mg/m², 55 mg/m², 60 mg/m², 65 mg/m², 70 mg/m², 75 mg/m², 80 mg/m², 85 mg/m², 90 mg/m², or 95 mg/m². In the invention, 60 mg/m² to 85 mg/m² of oxaliplatin is administered to the human patient.

Dose modifications may be made to methods of administering the combination treatment described herein as a result of adverse events, include hematological and non-hematological adverse events.

In some embodiments, methods of administering the combination treatment described herein to patients having one or more characteristics can include reducing or otherwise modifying the dose of MM-398 administered according to the embodiments herein. In some embodiments, the dose of MM-398 is modified according to Table 1.

**Table 1A: Examples of Dose Modifications for MM-398 (salt)**

| **Toxicity NCI CTCAE v4.0** | **Occurrence** | **MM-398 adjustment in patients receiving** | **Patients homozygous for UGT1A1*28 without previous increase to** |
|---|---|---|---|
| | | **60 mg/m^{2‡} (salt)** | **60 mg/m² (salt)** |
| **Grade 3 or 4 adverse reactions** | Withhold MM-398. | | |
| | Initiate loperamide for late onset diarrhea of any severity. | | |
| | Administer intravenous or subcutaneous atropine 0.25 to 1 mg (unless clinically contraindicated) for early onset diarrhea of any severity. | | |
| | Upon recovery to ≤ Grade 1 or baseline grade resume MM-398 at: | | |
| | First | 45 mg/m² | 35 mg/m² |
| | Second | 35 mg/m² | 30 mg/m² |
| | Third | Discontinue MM-398 | Discontinue MM-398 |
| **Interstitial Lung Disease** | First | Discontinue MM-398 | Discontinue MM-398 |
| **Anaphylactic Reaction** | First | Discontinue MM-398 | Discontinue MM-398 |

In some embodiments, the first, second or any subsequent dose of MM-398 can be reduced by 20-30% (including dose reductions of 20%, 25% and/or 30%) in response to patient tolerability considerations such as an adverse reaction to a first or subsequent dose of MM-398 and/or other antineoplastic agent, and/or identifying a patient as being homozygous for the UGT1A1*28 allele. In some embodiments, the second or subsequent dose of MM-398 is reduced by about 20%, 25% or 30% (e.g., a dose reduction from 60 mg/m2 to . In some embodiments, the dose of MM-398 is reduced by 25%. In some embodiments, the dose of MM-398 is reduced by 30%. In some embodiments, the reduced dose of MM-398 is in a range starting from 30 mg/m² to (and including) 55 mg/m². In some embodiments, the dose of MM-398 is reduced to 60 mg/m². In some embodiments, the dose of MM-398 is reduced to 45 mg/m². In some embodiments, the dose of MM-398 is reduced to 35 mg/m².

Other dose reduction schedules are provided Tables 1B-1E below. When the starting (initial) dose of MM-398 is 60 mg/m², 5FU 2400mg/m², LV(l+d) 400mg/m² and Oxaliplatin is either 85mg/m2 OR 60mg/m2, then the first dose reduction in response to a grade III or IV hematotoxicity is preferably a 25% dose reduction for each of the MM-398, 5-FU and Oxaliplatin doses for each administration of the antineoplastic therapy. For persistent toxicities despite the first dose reduction, an additional 25% dose reduction in each of the antineoplastic agents of MM-398, 5-fluorouracil and oxaliplatin is preferred. Further toxicity will then lead to discontinuation of treatment in some instances. For non-hematologic toxicities, the same dose reduction schema can be followed as for hematotoxicity, except for the specific toxicities associated with the drug (ie 5FU hand foot syndrome, and oxaliplatin neuropathy) which can be selected based on the medically appropriate dose for the patient.

**Table 1B Examples of Reduced Doses of MM-398 and oxaliplatin**

| Dose | MM-398 (mg/m2) (salt) | Oxaliplatin (mg/m2) | 5-fluorouracil (5FU) (mg/m2) |
|---|---|---|---|
| Initial | 60 | 60 | 2400 |
| First Reduction | 45 | 45 | 1800 |
| Second Reduction | 35 | 35 | 1350 |

**Table 1C Examples of Reduced Doses of MM-398 and oxaliplatin**

| Dose | MM-398 (mg/m2) (salt) | Oxaliplatin (mg/m2) | 5-fluorouracil (5FU) (mg/m2) |
|---|---|---|---|
| Initial | 60 | 80 | 2400 |
| First Reduction | 45 | 60 | 1800 |
| Second Reduction | 35 | 45 | 1350 |

**Table 1D Examples of Reduced Doses of MM-398 and oxaliplatin**

| Dose | MM-398 (mg/m2) (salt) | Oxaliplatin (mg/m2) | 5-fluorouracil (5FU) (mg/m2) |
|---|---|---|---|
| Initial | 60 | 60 | 2400 |
| First Reduction | 45 | 45 | 2400 |
| Second Reduction | 35 | 35 | 1800 |

**Table 1E Examples of Reduced Doses of MM-398 and oxaliplatin**

| Dose | MM-398 (mg/m2) (salt) | Oxaliplatin (mg/m2) | 5-fluorouracil (5FU) (mg/m2) |
|---|---|---|---|
| Initial | 60 | 80 | 2400 |
| First Reduction | 45 | 60 | 2400 |
| Second Reduction | 35 | 45 | 1800 |

In some embodiments, methods of administering the combination treatment described herein to patients having one or more characteristics can include reducing or otherwise modifying the dose of Oxaliplatin administered according to the embodiments herein. In some embodiments, the dose of Oxaliplatin is reduced by 20-30%. In some embodiments, the dose of Oxaliplatin is reduced by 20%. In some embodiments, the dose of Oxaliplatin is reduced by 25%. In some embodiments, the dose of Oxaliplatin is reduced by 30%. In some embodiments, the reduced dose of Oxaliplatin is in a range from 30 mg/m² to 75 mg/m². In some embodiments, the dose of Oxaliplatin is reduced to 75 mg/m². In some embodiments, the dose of Oxaliplatin is reduced to 65 mg/m². In some embodiments, the dose of Oxaliplatin is reduced to 60 mg/m². In some embodiments, the dose of Oxaliplatin is reduced to 45 mg/m². In some embodiments, the dose of Oxaliplatin is reduced to 45 mg/m². In some embodiments, the dose of Oxaliplatin is reduced to 34 mg/m².

In some embodiments, methods of administering the combination treatment described herein to patients having one or more characteristics can include reducing or otherwise modifying the dose of 5-fluorouracil administered according to the embodiments herein. In some embodiments, the dose of 5-fluorouracil is reduced by 20-30%. In some embodiments, the dose of 5-fluorouracil is reduced by 20%. In some embodiments, the dose of 5-fluorouracil is reduced by 25%. In some embodiments, the dose of 5-fluorouracil is reduced by 30%. In some embodiments, the reduced dose of 5-fluorouracil is in a range from 1000 mg/m² to 1800 mg/m². In some embodiments, the dose of 5-fluorouracil is reduced to 1800 mg/m². In some embodiments, the dose of 5-fluorouracil is reduced to 1350 mg/m². In some embodiments, the dose of 5-fluorouracil is reduced to 1200 mg/m².

In some embodiments, methods of administering the combination treatment described herein to patients having one or more characteristics can include further reducing or otherwise modifying the dose of MM-398, Oxaliplatin and/or 5-fluorouracil administered according to the embodiments herein.

In some embodiments, methods of administering the combination treatment described herein to patients having one or more characteristics can include reducing or otherwise modifying the dose of more than one of MM-398, Oxaliplatin and 5-fluorouracil administered according to the embodiments herein.

Additional dose modifications for MM-398, Oxaliplatin and/or 5-fluorouracil can be found in the respective Package Inserts.

In one embodiment, the method of administering the combination treatment comprises 34, 45, or 60 mg/m² of liposomal irinotecan, 34, 42, 45, 60 or 85 mg/m² oxaliplatin, 200 mg/m² of (I)-form of leucovorin or 400 mg/m² of the (I+d) racemic form of leucovorin, and 1,200, 1,350, 1,800 or 2,400 mg/m² 5-fluorouracil to treat the metastatic adenocarcinoma of the pancreas in the human patient.

Thus, in some embodiments, the method of administering the combination treatment to treat the metastatic adenocarcinoma of the pancreas in the human patient comprises:
(A) (i) 35 mg/m² of liposomal irinotecan, 35 mg/m² oxaliplatin, 200 mg/m² (I)-form or 400 mg/m² racemic leucovorin, and 1,200mg/m² 5-FU; (ii) 35 mg/m² of liposomal irinotecan, 35 mg/m² oxaliplatin, 200 mg/m² (I)-form or 400 mg/m² racemic leucovorin, and 1,350mg/m² 5-FU; (iii) 35 mg/m² of liposomal irinotecan, 35 mg/m² oxaliplatin, 200 mg/m² (I)-form or 400 mg/m² racemic leucovorin, and 1,800mg/m² 5-FU; (iv) 35 mg/m² of liposomal irinotecan, 35 mg/m² oxaliplatin, 200 mg/m² (I)-form or 400 mg/m² racemic leucovorin, and 2,400mg/m² 5-FU; (v) 35 mg/m² of liposomal irinotecan, 45 mg/m² oxaliplatin, 200 mg/m² (l)-form or 400 mg/m² racemic leucovorin, and 1,200mg/m² 5-FU; (vi) 35 mg/m² of liposomal irinotecan, 45 mg/m² oxaliplatin, 200 mg/m² (I)-form or 400 mg/m² racemic leucovorin, and 1,350mg/m² 5-FU; (vii) 35 mg/m² of liposomal irinotecan, 45 mg/m² oxaliplatin, 200 mg/m² (I)-form or 400 mg/m² racemic leucovorin, and 1,800mg/m² 5-FU; (viii) 35 mg/m² of liposomal irinotecan, 45 mg/m² oxaliplatin, 200 mg/m² (I)-form or 400 mg/m² racemic leucovorin, and 2,400mg/m² 5-FU; (ix) 35 mg/m² of liposomal irinotecan, 45 mg/m² oxaliplatin, 200 mg/m² (I)-form or 400 mg/m² racemic leucovorin, and 1,200mg/m² 5-FU; (x) 35 mg/m² of liposomal irinotecan, 45 mg/m² oxaliplatin, 200 mg/m² (I)-form or 400 mg/m² racemic leucovorin, and 1,350mg/m² 5-FU; (xi) 35 mg/m² of liposomal irinotecan, 45 mg/m² oxaliplatin, 200 mg/m² (l)-form or 400 mg/m² racemic leucovorin, and 1,800mg/m² 5-FU; (xii) 35 mg/m² of liposomal irinotecan, 45 mg/m² oxaliplatin, 200 mg/m² (I)-form or 400 mg/m² racemic leucovorin, and 2,400mg/m² 5-FU; (xiii) 35 mg/m² of liposomal irinotecan, 60 mg/m² oxaliplatin, 200 mg/m² (I)-form or 400 mg/m² racemic leucovorin, and 1,200mg/m² 5-FU; (xiv) 35 mg/m² of liposomal irinotecan, 60 mg/m² oxaliplatin, 200 mg/m² (l)-form or 400 mg/m² racemic leucovorin, and 1,350mg/m² 5-FU; (xv) 35 mg/m² of liposomal irinotecan, 60 mg/m² oxaliplatin, 200 mg/m² (I)-form or 400 mg/m² racemic leucovorin, and 1,800mg/m² 5-FU; (xvi) 35 mg/m² of liposomal irinotecan, 60 mg/m² oxaliplatin, 200 mg/m² (I)-form or 400 mg/m² racemic leucovorin, and 2,400mg/m² 5-FU; (xvii) 35 mg/m² of liposomal irinotecan, 85 mg/m² oxaliplatin, 200 mg/m² (l)-form or 400 mg/m² racemic leucovorin, and 1,200mg/m² 5-FU; (xviii) 35 mg/m² of liposomal irinotecan, 85 mg/m² oxaliplatin, 200 mg/m² (I)-form or 400 mg/m² racemic leucovorin, and 1,350mg/m² 5-FU; (xix) 35 mg/m² of liposomal irinotecan, 85 mg/m² oxaliplatin, 200 mg/m² (l)-form or 400 mg/m² racemic leucovorin, and 1,800mg/m² 5-FU; or (xx) 35 mg/m² of liposomal irinotecan, 85 mg/m² oxaliplatin, 200 mg/m² (I)-form or 400 mg/m² racemic leucovorin, and 2,400mg/m² 5-FU; (B) (i) 45 mg/m² of liposomal irinotecan, 35 mg/m² oxaliplatin, 200 mg/m² (I)-form or 400 mg/m² racemic leucovorin, and 1,200mg/m² 5-FU; (ii) 45 mg/m² of liposomal irinotecan, 35 mg/m² oxaliplatin, 200 mg/m² (I)-form or 400 mg/m² racemic leucovorin, and 1,350mg/m² 5-FU; (iii) 45 mg/m² of liposomal irinotecan, 35 mg/m² oxaliplatin, 200 mg/m² (I)-form or 400 mg/m² racemic leucovorin, and 1,800mg/m² 5-FU; (iv) 45 mg/m² of liposomal irinotecan, 35 mg/m² oxaliplatin, 200 mg/m² (I)-form or 400 mg/m² racemic leucovorin, and 2,400mg/m² 5-FU; (v) 45 mg/m² of liposomal irinotecan, 45 mg/m² oxaliplatin, 200 mg/m² (I)-form or 400 mg/m² racemic leucovorin, and 1,200mg/m² 5-FU; (vi) 45 mg/m² of liposomal irinotecan, 45 mg/m² oxaliplatin, 200 mg/m² (I)-form or 400 mg/m² racemic leucovorin, and 1,350mg/m² 5-FU; (vii) 45 mg/m² of liposomal irinotecan, 45 mg/m² oxaliplatin, 200 mg/m² (I)-form or 400 mg/m² racemic leucovorin, and 1,800mg/m² 5-FU; (viii) 45 mg/m² of liposomal irinotecan, 45 mg/m² oxaliplatin, 200 mg/m² (l)-form or 400 mg/m² racemic leucovorin, and 2,400mg/m² 5-FU; (ix) 45 mg/m² of liposomal irinotecan, 45 mg/m² oxaliplatin, 200 mg/m² (I)-form or 400 mg/m² racemic leucovorin, and 1,200mg/m² 5-FU; (x) 45 mg/m² of liposomal irinotecan, 45 mg/m² oxaliplatin, 200 mg/m² (I)-form or 400 mg/m² racemic leucovorin, and 1,350mg/m² 5-FU; (xi) 45 mg/m² of liposomal irinotecan, 45 mg/m² oxaliplatin, 200 mg/m² (l)-form or 400 mg/m² racemic leucovorin, and 1,800mg/m² 5-FU; (xii) 45 mg/m² of liposomal irinotecan, 45 mg/m² oxaliplatin, 200 mg/m² (I)-form or 400 mg/m² racemic leucovorin, and 2,400mg/m² 5-FU; (xiii) 45 mg/m² of liposomal irinotecan, 60 mg/m² oxaliplatin, 200 mg/m² (I)-form or 400 mg/m² racemic leucovorin, and 1,200mg/m² 5-FU; (xiv) 45 mg/m² of liposomal irinotecan, 60 mg/m² oxaliplatin, 200 mg/m² (l)-form or 400 mg/m² racemic leucovorin, and 1,350mg/m² 5-FU; (xv) 45 mg/m² of liposomal irinotecan, 60 mg/m² oxaliplatin, 200 mg/m² (I)-form or 400 mg/m² racemic leucovorin, and 1,800mg/m² 5-FU; (xvi) 45 mg/m² of liposomal irinotecan, 60 mg/m² oxaliplatin, 200 mg/m² (I)-form or 400 mg/m² racemic leucovorin, and 2,400mg/m² 5-FU; (xvii) 45 mg/m² of liposomal irinotecan, 85 mg/m² oxaliplatin, 200 mg/m² (l)-form or 400 mg/m² racemic leucovorin, and 1,200mg/m² 5-FU; (xviii) 45 mg/m² of liposomal irinotecan, 85 mg/m² oxaliplatin, 200 mg/m² (I)-form or 400 mg/m² racemic leucovorin, and 1,350mg/m² 5-FU; (xix) 45 mg/m² of liposomal irinotecan, 85 mg/m² oxaliplatin, 200 mg/m² (l)-form or 400 mg/m² racemic leucovorin, and 1,800mg/m² 5-FU; or (xx) 45 mg/m² of liposomal irinotecan, 85 mg/m² oxaliplatin, 200 mg/m² (I)-form or 400 mg/m² racemic leucovorin, and 2,400mg/m² 5-FU; or (C) (i) 60 mg/m² of liposomal irinotecan, 35 mg/m² oxaliplatin, 200 mg/m² (I)-form or 400 mg/m² racemic leucovorin, and 1,200mg/m² 5-FU; (ii) 60 mg/m² of liposomal irinotecan, 35 mg/m² oxaliplatin, 200 mg/m² (I)-form or 400 mg/m² racemic leucovorin, and 1,350mg/m² 5-FU; (iii) 60 mg/m² of liposomal irinotecan, 35 mg/m² oxaliplatin, 200 mg/m² (I)-form or 400 mg/m² racemic leucovorin, and 1,800mg/m² 5-FU; (iv) 60 mg/m² of liposomal irinotecan, 35 mg/m² oxaliplatin, 200 mg/m² (I)-form or 400 mg/m² racemic leucovorin, and 2,400mg/m² 5-FU; (v) 60 mg/m² of liposomal irinotecan, 45 mg/m² oxaliplatin, 200 mg/m² (I)-form or 400 mg/m² racemic leucovorin, and 1,200mg/m² 5-FU; (vi) 60 mg/m² of liposomal irinotecan, 45 mg/m² oxaliplatin, 200 mg/m² (I)-form or 400 mg/m² racemic leucovorin, and 1,350mg/m² 5-FU; (vii) 60 mg/m² of liposomal irinotecan, 45 mg/m² oxaliplatin, 200 mg/m² (I)-form or 400 mg/m² racemic leucovorin, and 1,800mg/m² 5-FU; (viii) 60 mg/m² of liposomal irinotecan, 45 mg/m² oxaliplatin, 200 mg/m² (I)-form or 400 mg/m² racemic leucovorin, and 2,400mg/m² 5-FU; (ix) 60 mg/m² of liposomal irinotecan, 45 mg/m² oxaliplatin, 200 mg/m² (I)-form or 400 mg/m² racemic leucovorin, and 1,200mg/m² 5-FU; (x) 60 mg/m² of liposomal irinotecan, 45 mg/m² oxaliplatin, 200 mg/m² (I)-form or 400 mg/m² racemic leucovorin, and 1,350mg/m² 5-FU; (xi) 60 mg/m² of liposomal irinotecan, 45 mg/m² oxaliplatin, 200 mg/m² (l)-form or 400 mg/m² racemic leucovorin, and 1,800mg/m² 5-FU; (xii) 60 mg/m² of liposomal irinotecan, 45 mg/m² oxaliplatin, 200 mg/m² (I)-form or 400 mg/m² racemic leucovorin, and 2,400mg/m² 5-FU; (xiii) 60 mg/m² of liposomal irinotecan, 60 mg/m² oxaliplatin, 200 mg/m² (l)-form or 400 mg/m² racemic leucovorin, and 1,200mg/m² 5-FU; (xiv) 60 mg/m² of liposomal irinotecan, 60 mg/m² oxaliplatin, 200 mg/m² (I)-form or 400 mg/m² racemic leucovorin, and 1,350mg/m² 5-FU; (xv) 60 mg/m² of liposomal irinotecan, 60 mg/m² oxaliplatin, 200 mg/m² (l)-form or 400 mg/m² racemic leucovorin, and 1,800mg/m² 5-FU; (xvi) 60 mg/m² of liposomal irinotecan, 60 mg/m² oxaliplatin, 200 mg/m² (I)-form or 400 mg/m² racemic leucovorin, and 2,400mg/m²5-FU; (xvii) 60 mg/m² of liposomal irinotecan, 85 mg/m² oxaliplatin, 200 mg/m² (l)-form or 400 mg/m² racemic leucovorin, and 1,200mg/m² 5-FU; (xviii) 60 mg/m² of liposomal irinotecan, 85 mg/m² oxaliplatin, 200 mg/m² (I)-form or 400 mg/m² racemic leucovorin, and 1,350mg/m² 5-FU; (xix) 60 mg/m² of liposomal irinotecan, 85 mg/m² oxaliplatin, 200 mg/m² (l)-form or 400 mg/m² racemic leucovorin, and 1,800mg/m² 5-FU; or(xx) 60 mg/m² of liposomal irinotecan, 85 mg/m² oxaliplatin, 200 mg/m² (I)-form or 400 mg/m² racemic leucovorin, and 2,400mg/m² 5-FU.

Liposomal irinotecan is preferably administered intravenously, in combination with oxaliplatin, 5-fluorouracil (5-FU) and leucovorin. In one embodiment, liposomal irinotecan is administered prior to oxaliplatin, 5-FU and leucovorin. In another embodiment, leucovorin is administered prior to 5-FU. In another embodiment, the MM-398 liposomal irinotecan is administered followed by administration of the oxaliplatin, followed by administration of the leucovorin, and followed by the administration of the 5-fluorouracil. In certain embodiments, the liposomal irinotecan is administered to the patient intravenously over 90 minutes. In another embodiment, the oxaliplatin is administered to the patient intravenously over 120 minutes. In another embodiment, 5-FU is administered intravenously over 46 hours. In one embodiment, the oxaliplatin is administered from about 6 to about 72 hours after administration of the liposomal irinotecan. In another embodiment, the oxaliplatin is administered for example, 6 hours, 12 hours, 24 hours, 36 hours, 48 hours, 60 hours, or 72 hours, after administration of the liposomal irinotecan. In another embodiment, leucovorin is administered intravenously over 30 minutes. In various embodiments the liposomal irinotecan is MM-398. In various embodiments, the human patient with metastatic pancreatic cancer is pre-medicated with dexamethasone and a 5-HT3 antagonist or other anti-emetic prior to administering the MM-398 liposomal irinotecan, and other active agents.

The invention provides liposomal irinotecan for use in methods of treating metastatic adenocarcinoma of the pancreas in a human patient who has not previously received chemotherapy to treat the metastatic adenocarcinoma of the pancreas, the method comprising administering to the patient liposomal irinotecan, also referred to as MM-398 (e.g., irinotecan sucrose octasulfate salt liposome injection) in combination with oxaliplatin, leucovorin and 5-FU. In the invention, 60 mg/m² of MM-398 liposomal irinotecan is administered to the patient.

In the invention, the amount of oxaliplatin administered is from 60 mg/m² to 85 mg/m², for example 60 mg/m², 75 mg/m², or 85 mg/m².

In the invention, the leucovorin is administered at a dosage of 400 mg/m² of the (I + d) racemic form, or 200 mg/m² of the (l) form.

In the invention, the amount of 5-FU administered is 2,400 mg/m².

In some embodiments, the MM-398 liposomal irinotecan, oxaliplatin, leucovorin, and 5-FU are administered at least once, such as wherein the MM-398, oxaliplatin, leucovorin, and 5-FU are administered on days 1 and 15 of a 28-day cycle.

In some embodiments, multiple cycles are administered.

In some embodiments, the oxaliplatin is administered to the patient prior to the leucovorin, such as wherein the leucovorin is administered to the patient prior to the 5-FU, optionally wherein the MM-398 liposomal irinotecan is administered to the patient prior to the oxaliplatin, leucovorin, and 5-FU.

In some embodiments, the MM-398 is administered over 90 minutes, followed by administration of the oxaliplatin over 120 minutes, followed by administration of the leucovorin over 30 minutes, followed by the administration of the 5-FU over 46 hours.

### Examples

### Example 1: In vitro pancreatic cancer cell exposure to topoisomerase 1 inhibitor

Simulated tumor exposure of SN-38 in patients administered with free irinotecan or MM-398 were shown in Figure 1A. MM-398 is shown to result in prolonged SN-38 duration in tumors compared to free irinotecan (CPT-11). The effect of various SN-38 durations on cell growth inhibition was studied in a panel of pancreatic cell lines (AsPC-1, BxPC-3, Capan-2, CFPAC-1, and MiaPaCa-2). Figure 1B illustrates the in vitro conditions for mimicking this clinically comparable SN-38 exposure of the 2 drugs, where cells exposed to SN-38 at high concentrations for a short period of time approximates for free irinotecan, and at low concentrations for a long period of time for MM-398. The results and experimental conditions are summarized in Figure 1C. For example, cells incubated with 139 nM of SN-38 for 144h vs. 417 nM for 24h have similar SN-38 tumor exposure ratios of MM-398 vs. free irinotecan in patient tumors. Under these clinically relevant conditions, prolonged exposure (i.e. MM-398) primarily resulted in more pancreatic cancer cell growth inhibition compared to short exposure at high concentrations (i.e. free irinotecan). Similar results were also obtained when SN-38 were combined with 5-FU or oxaliplatin, demonstrating that prolonged exposure also led to increased cell growth inhibition when combined with these other chemotherapeutics agents that are used in the FOLFIRINOX regimen.

### Example 2: Evaluation of in vivo tolerability and efficacy of combination therapies in an animal model

### BxPC-3 and CFPAC-1 mouse xenograft studies (efficacy):

Tissue culture: BxPC-3 cells were cultured in RPMI growth media supplemented with 10% FBS and 1% penicillin/streptomycin. CFPAC-1 cells were also cultured in RPMI growth media supplemented with 10% FBS and 1% penicillin/streptomycin.

Animals: Experiments were performed according to approved guidelines. Female NOD.scid mice were obtained from Charles River Laboratories (Wilmington, MA). BxPC-3 or CFPAC-1 cells were inoculated into the right hind flank at 5e6 cells in a total volume of 50 uL per mouse. Eight animals were treated per group, unless otherwise indicated. Animals were randomized and dosing initiated when tumors reached an average volume of 200-250 mm³ (range 100-400 mm³), unless otherwise indicated.

Treatment efficacy: MM-398, irinotecan and oxaliplatin were administered intravenously. 5-FU was administered intraperitoneally. Administration of the indicated doses of each agent was initiated when tumors reached an average volume of 200-250 mm³ and continued for a total of 4 weekly doses. Tumor volumes were measured weekly until tumors reached 1000-2000 mm³, as indicated, animals were in poor general health, or 2 weeks post post-final dose.

### PDX19015 mouse xenograft study (efficacy and tolerability):

Animals: Experiments were performed according to approved guidelines. Female CB.17 SCID mice were obtained from Roswell Park Cancer Institute (Buffalo, NY), initially at 6-8 weeks of age. Per treatment group, 8 animals were treated, unless otherwise indicated. Tumor pieces were derived from donor mice and engrafted subcutaneously. Animals were randomized and dosing initiated when tumors reached an average volume of 200-250 mm³ (range 100-400 mm³), unless otherwise indicated.

Treatment efficacy: MM-398, irinotecan and oxaliplatin were administered intravenously. 5-FU was administered intraperitoneally. Administration of the indicated doses of each agent was initiated when tumors reached an average volume of 200-250 mm³ and continued for a total of 4 weekly doses. Tumor volumes were measured twice weekly during the dosing cycle, then once weekly until tumors reached 1000-2000 mm³, as indicated, animals were in poor general health, or 100 days post-first dose. Tolerability: Mouse weights were measured once weekly to monitor treatment tolerability. Mice were euthanized when body weight declined to ≥20% below baseline, or they exhibited overt signs of poor general health.

### Delayed dosing of oxaliplatin:

Animals: Experiments were performed according to approved guidelines. Female CD-1 mice were obtained from Charles River Laboratories (Wilmington, MA). Tolerability studies were performed in naïve (non-tumor-bearing) mice. Three animals were treated per group.

Treatment tolerability: Agents were administered intravenously at their pre-defined maximum tolerated doses (MM-398, 50mg/kg; oxaliplatin, 17mg/kg). Each drug was administered individually, or in combination. Combinations were given in one of 3 independent dosing schedules: coinjection (drugs administered simultaneously), MM-398 given on day 1 and oxaliplatin given on day 2 (24h delay), or MM-398 given on day 1 and oxaliplatin given on day 4 (72h delay). A single administration of each drug was given. Mouse body weights were measured daily for up to 2 weeks post-treatment. Mice were euthanized when body weight declined to ≥20% below baseline, they exhibited overt signs of poor general health, or at 2 weeks post-treatment (end of study).

Measurement of hematologic and liver toxicities: At the end of study, terminal bleeds were performed for each mouse via cardiac puncture. Hematologic function (blood cell count) was measured by Hemavet (Drew Scientific, Miami Lakes, FL), according to manufacturer's protocol. Liver function (enzyme levels) was measured by CatalystDx (Idexx Laboratories, Westbrook, ME) according to the manufacturer's protocol.

### Example 3: Treatment of Pancreatic Cancer

As schematically shown in Figure 12, the present study is an open-label, phase 2 comparative study to assess the safety, tolerability, and efficacy of MM-398 in combination with other anticancer therapies, compared to nab-paclitaxel + gemcitabine, in patients with metastatic pancreatic adenocarcinoma who have not received prior chemotherapy. This study assesses the following regimens: (1) MM-398 + 5-FU/LV + oxaliplatin (Arm 1), (2) MM-398 + 5-FU/LV (Arm 2) and (3) nab-paclitaxel + gemcitabine (Arm 3).

This phase 2 study evaluates the preliminary safety and efficacy of MM-398 + 5-FU/LV with or without oxaliplatin versus nab-paclitaxel + gemcitabine in patients with previously untreated mPAC. The study may also provide important information on the impact of MM-398 combination treatment on patient HRQL and identify potential biomarkers of response.

In the study, MM-398 is administered instead of conventional irinotecan to improve the safety, tolerability, and ultimately efficacy of a FOLFIRINOX regimen. The addition of oxaliplatin to the NAPOLI-1 regimen is included to increase DNA damage and potentiate efficacy. Further, due to the MM-398 prolonged PK properties and sustained tumor exposure, using MM-398 instead of conventional irinotecan is designed to further improve upon the efficacy of FOLFIRINOX.

A modified triplet combination regimen of liposomal irinotecan, oxaliplatin, 5-fluorouracil (5-FU)/leucovorin is provided herein, whereby no bolus of 5-FU will be administered. The target dose of oxaliplatin (60-85 mg/m²) is evaluated in the Arm 1 combination regimen with the continuous infusion dose of 5-FU (excluding the bolus), and the every 2 week dose of MM-398 previously shown to be tolerable and efficacious in combination with 5-FU. Note that with MM-398 dosing, the Cₘₐₓ of SN-38 is expected to be lower than would be expected for standard dosing with free irinotecan.

The study is conducted in two parts, as illustrated in the schematic of Figure 12: 1) a safety run-in of the MM-398 + 5-FU/LV + oxaliplatin regimen, and 2) a randomized, efficacy study of the MM-398 + 5-FU/LV + oxaliplatin regimen, the MM-398 + 5-FU/LV combination that previously demonstrated efficacy in the Phase 3 NAPOLI-1 trial (i.e. the NAPOLI regimen), and a nab-paclitaxel + gemcitabine control arm.

### Part 1:

Part 1 consists of an open-label safety run-in of the combination regimen in Arm 1: MM-398 + 5-FU/LV + oxaliplatin. The Arm 2 and Arm 3 regimens have established doses, and MM-398 + 5-FU/LV has been demonstrated tolerable, yielding antitumor responses in a Phase 3 study of patients with relapsed metastatic pancreatic cancer, and therefore was not included in this part of the study. The safety run-in enrolls small cohorts of patients following a traditional 3 + 3 dose escalation design in order to confirm the target dose of oxaliplatin. Dose limiting toxicities (DLTs) are evaluated during the first cycle of treatment (i.e. 28 days per cycle; or 14 days after the 2^{nd} dose of study treatment if there is a treatment delay in cohorts of patients to determine if the target combination dose is tolerable (note: the target combination dose is based on the established dose of the FOLFIRINOX regimen)). If there are no DLTs within the safety evaluation period, then the subsequent cohort is initiated following agreement between the Investigators, Medical Monitor, and the Sponsor. If one DLT occurs, then the cohort is expanded to 6 patients. If 2 or more patients have DLTs within a given dose level, that dose is considered to exceed the safety and tolerability criteria of the combination, and the dose is not be escalated further; however, lower doses can be explored. The Part 2 dose is then defined as the next lower dose level in which 6 patients were treated and ≤ 1 patient experienced a toxicity that qualifies as a DLT.

Additionally, UGT1A1*28 allele status is considered when evaluating DLTs. Based on previous experience with irinotecan, individuals who are homozygous for the UGT1A1*28 allele (UGT1A1 7/7 genotype) are at increased risk for neutropenia following initiation of irinotecan treatment. According to the prescribing information for irinotecan, in a study of 66 patients who received single-agent irinotecan (350 mg/m² once every-3-weeks), the incidence of grade 4 neutropenia in patients homozygous for the UGT1A1*28 allele was as high as 50%, and in patients heterozygous for this allele (UGT1A1 6/7 genotype) the incidence was 12.5%. Importantly, no grade 4 neutropenia was observed in patients homozygous for the wild-type (WT) allele (UGT1A1 6/6 genotype). In other studies, a lower prevalence of accompanying life threatening neutropenia is described (for details refer to the prescribing information for irinotecan). Population PK studies of MM-398 have not identified a relationship between UGT1A1*28 homozygosity and increased SN-38 exposure (see Investigator Brochure). In a Phase I study, no differences in toxicity were seen in cohorts of heterozygous or WT patients, and DLTs of diarrhea with or without accompanying dehydration or fatigue, were seen in both cohorts. For these reasons, and because the prevalence of UGT1A1*28 homozygosity is relatively low, testing results are not required prior to the first dose of MM-398 on this study and the starting dose for all patients will be 80 mg/m². However, if patients are known to be homozygous for UGT1A1*28, the dose of MM-398 may be reduced as described herein.

### Part 2:

Part 2 consists of an open-label, randomized, Phase 2 study where patients will be randomized to treatment (1:1:1) to either MM-398 + 5-FU/LV + oxaliplatin, MM-398 + 5-FU/LV, or nab-paclitaxel + gemcitabine. The randomization is stratified based on region (East Asia vs. rest of the world) and performance status (ECOG 0 vs. 1).

The following adverse events are common (≥ 40%) with past oxaliplatin treatment in combination with 5-FU/LV and are to be expected with the MM-398-containing combination regimen: peripheral sensory neuropathy, neutropenia, thrombocytopenia, anemia, nausea, increases in transaminases and alkaline phosphatase, diarrhea, fatigue, emesis, and stomatitis. Additional adverse events may be anticipated, as described in the package insert for oxaliplatin, including allergic and anaphylactic reactions. In a Phase 3 study of the FOLFIRINOX combination, the most common (> 5%) Grade 3-4 adverse events were: neutropenia, fatigue, vomiting, diarrhea, thrombocytopenia, sensory neuropathy, anemia, elevated alanine aminotransferase (ALT) level, thromboembolism, and febrile neutropenia. Considering these expected toxicities, Arm 1 is evaluated for safety and tolerability in Part 1 of the study as described below.

A dose of oxaliplatin of 85 mg/m² is the target dose for Part 2 of this study. The purpose of Part 1 is to confirm whether this dose is compatible when MM-398 is used instead of conventional irinotecan. In case there are any unexpected toxicities, 3 to 6 patients are initially treated at a lower dose of oxaliplatin (60 mg/m², see Table 1) prior to administration of oxaliplatin at the highest proposed dose of 85 mg/m². The dose of the triplet combination to be administered in Part 2 of the study is defined as the highest dose level at which a DLT is experienced by fewer than 2 patients in a cohort of 3 to 6 patients. If one patient experiences a treatment-related toxicity that qualifies as a DLT, up to 3 additional patients are enrolled at that dose level, for no more than 6 total patients per cohort. If no additional DLTs are observed, the dose escalation resumes. If a second patient experiences a treatment-related toxicity that qualifies as a DLT at that dose, that dose is considered to exceed the optimal safety and tolerability criteria of the combination. The dose to be used in Part 2 is then defined as the next lower dose level in which 6 patients were treated and ≤ 1 patient experienced a toxicity that qualifies as a DLT.

Dosing of patient cohorts begins at dose level -1 with planned escalation to dose level -2B (target dose), in which the dose for one of the three drugs is increased while the other two drugs will maintain a constant dose. If the -1 dose level is evaluated and deemed to be safe, escalation to the -2B dose level may be initiated. Any decisions to de-escalate, as well as enrollment at alternative doses following de-escalation, must be made according to the established decision process for dose escalation, as described herein. Planned dose escalation for the Arm 1 combination regimen is outlined in Table 2 below; additional details on dose administration as described herein in the section "Study Treatment".

**Table 2 Part 1 Dose Escalation Table (MM-398 + 5-FU/LV + oxaliplatin)**

| **Level** | **Oxaliplatin** | | **5-FU/LV** | | **MM-398 (nal-IRI)** | |
|---|---|---|---|---|---|---|
| | **Dose (mg/m²)^{a},** | **Dose Day^{c}** | **Dose (mg/m²)^{b}** | **Dose Day^{c}** | **Dose (mg/m²)** | **Dose Day^{c}** |
| -1 | 60 | 1, 15 | 2400/400 | 1, 15 | 60 | 1, 15 |
| -2B | 85 | 1, 15 | 2400/400 | 1, 15 | 60 | 1, 15 |

| | | | | | | |
|---|---|---|---|---|---|---|
| a First dose administration in conjunction with first dose of MM-398; oxaliplatin to be administered 2 hours after the completion of the nal-IRI infusion in Part 1. b 46 hour infusion, no bolus is given; leucovorin and 5-FU will be administered last, following the completion of the oxaliplatin infusion c Day indicated is part of a 28-day cycle | | | | | | |

### Arm 1: MM-398 + 5-FU/LV + Oxaliplatin

The order of the infusions to be administered in the clinic is as follows: MM-398 administered first, followed by oxaliplatin, then LV, followed by 5-FU.

In Part 1, patients receive the oxaliplatin infusion 2 hours after the completion of the MM-398 infusion. If no infusion reactions are seen, Part 2 patients can receive oxaliplatin directly after completion of the MM-398 infusion. If any grade 3 or higher infusion reactions are seen in Part 2 patients, the DSMB may elect to revert back to administration of oxaliplatin two hours after the completion of the MM-398 infusion.

### Arm 1 Premedication

All patients must be premedicated prior to MM-398 infusion, 5-FU/LV infusion, and oxaliplatin infusion with standard doses of dexamethasone and a 5-HT3 antagonist, or equivalent other anti-emetics according to standard institutional practices for irinotecan, 5-FU, and oxaliplatin administration, or the Summary of Product Characteristics (SmPC) for sites located in the European Union (EU). Atropine may be prescribed prophylactically for patients who experienced acute cholinergic symptoms in the previous cycles.

### Arm 2: MM-398 + 5-FU/LV

The order of the infusions to be administered in the clinic will be as follows: MM-398 will be administered first, followed by LV, followed by 5-FU.

### Arm 2 Premedication

All patients must be premedicated prior to MM-398 infusion and 5-FU/LV infusion with standard doses of dexamethasone and a 5-HT3 antagonist, or equivalent other anti-emetics according to standard institutional practices for irinotecan and 5-FU administration, or the SmPC for sites located in the EU. Atropine may be prescribed prophylactically, according to standard institutional practices, for patients who experienced acute cholinergic symptoms in the previous cycles.

### Doses and Administration of MM-398 (Arms 1 and 2)

MM-398 is administered by intravenous (IV) infusion over 90 minutes (±10 minutes) every two weeks. The first cycle Day 1 is a fixed day; subsequent doses should be administered on the first day of each cycle +/- 2 days.

Prior to administration, the appropriate dose of MM-398 must be diluted in 5% Dextrose Injection solution (D5W) or normal saline to a final volume of 500 mL. Care should be taken not to use in-line filters or any diluents other than D5W or normal saline. MM-398 can be administered at a rate of up to 1 mL/sec (30 mg/sec).

The actual dose of MM-398 to be administered will be determined by calculating the patient's body surface area at the beginning of each cycle. A +/- 5% variance in the calculated total dose will be allowed for ease of dose administration. Since MM-398 vials are single-use vials, site staff must not store any unused portion of a vial for future use and they must discard unused portions of the product.

### Doses and Administration of 5-FU and Leucovorin (Arms 1 and 2)

Leucovorin is administered at a dose of 400 mg/m² of the (I + d)- racemic form, or (l) form 200 mg/m², as an IV infusion over 30 minutes (±5 minutes), on Days 1 and 15 of each 28-day cycle
5-FU is administered at a dose of 2400 mg/m² as an IV infusion over 46-hours (±60 minutes), on Days 1 and 15 of each 28-day cycle
Leucovorin should be reconstituted per the instructions on the package insert, SmPC or standard institutional guidelines for reconstitution of leucovorin.
Leucovorin should be administered prior to the 5-FU infusion (on Arm 1, leucovorin will be given concurrently with oxaliplatin). Actual dose of 5-FU and leucovorin to be administered is determined by calculating the patient's body surface area prior to each cycle. A +/- 5% variance in the calculated total dose will be allowed for ease of dose administration.

### Doses and Administration of Oxaliplatin (Arm 1 only)

In Part 1, oxaliplatin is administered at increasing dose levels as indicated in Table 2 (from 60 mg/m² - 85 mg/m²), IV over 120 minutes (±10 minutes), on Days 1 and 15 of each 28-day cycle
In Part 2, oxaliplatin is administered at a dose of 85 mg/m², IV over 120 minutes (±10 minutes), on Days 1 and 15 of each 28-day cycle (if target dose is confirmed in accordance with methods described herein).

Oxaliplatin should be prepared according to the instructions on the package insert, SmPC or per standard institutional guidelines for preparation and administration of oxaliplatin.

Oxaliplatin should be administered following MM-398 infusion; in Part 1, the first 3 patients in Dose Level 1 begin the oxaliplatin infusion two hours after the completion of the MM-398 infusion. Actual dose of oxaliplatin to be administered is determined by calculating the patient's body surface area prior to each cycle. A +/- 5% variance in the calculated total dose is allowed for ease of dose administration.

### Arm 3: nab-Paclitaxel + Gemcitabine

The order of the infusions to be administered in the clinic is as follows: nab-paclitaxel will be administered first, followed by gemcitabine.

### Arm 3 Premedication

All patients receiving nab-paclitaxel and gemcitabine should be pre-medicated per the respective package inserts. If different institutional guidelines exist for premedication of weekly nab-paclitaxel and/or gemcitabine, the investigator should use their standard practice or the SmPC for sites located in the EU.

### Doses and Administration of nab-Paclitaxel and Gemcitabine (Arm 3)

The nab-paclitaxel will be administered at 125 mg/m² IV over 35 minutes (±5 minutes), on Days 1, 8 and 15 of each 28-day cycle.

The gemcitabine will be administered at 1000 mg/m² IV over 30 minutes (±5 minutes), on Days 1, 8 and 15 of each 28-day cycle.

### Dose Limiting Toxicities (DLTs)

For MM-398 administered in combination with 5-FU/LV and oxaliplatin, the following adverse events are considered as dose limiting toxicities (DLTs) if they occur during the first cycle of treatment and are deemed related to the study treatment regimen:
- Grade 4 neutropenia or thrombocytopenia that does not resolve within 7 days despite optimal therapy (withholding study drug and administering concomitant medication, e.g. G-CSF administration for neutropenia);
- Grade 4 neutropenia complicated by fever ≥ 38.5 °C (i.e. febrile neutropenia) and/or Grade 3 neutropenia with infection;
- Inability to begin subsequent treatment course within 14 days of the scheduled date, due to drug-related toxicity; and
- Any grade 4 non-hematologic toxicity with the specific exclusion of: Fatigue/asthenia < 2 weeks in duration, increases in alkaline phosphatase level, nausea and vomiting ≤3 days duration (only considered dose limiting if they last > 72 hours after treatment with an optimal anti-emetic regimen), and diarrhea ≤3 days duration (only considered dose limiting if diarrhea lasts > 72 hours after treatment with an optimal anti-diarrheal regimen)

Any toxicity that is related to disease progression will not be considered a DLT.

The safety assessment period for purposes of DLT evaluation and dose escalation decisions is one cycle of treatment (i.e. 28 days; or 14 days after the 2nd dose of study treatment if there is a treatment delay according as described herein). The dose can escalate to the next level only after the safety data have been evaluated at the current dose level (once the last patient enrolled in the cohort completes the first cycle of treatment) and the criteria for safety and tolerability of the optimal dose have not been exceeded (see Section Part 2 dose definition). In addition, any drug-related toxicities of Grade 3 or higher that arise after Cycle 1 (if applicable) are assessed for their potential relationship to cumulative MM-398 or combination therapy doses and considered in the decision to escalate the dose. PK data may be available, but is not be required for decisions on dose escalation.

| Inclusion Criteria | | Exclusion Criteria | |
|---|---|---|---|
| In order for inclusion into the study, patients must have/be: | | Patients must meet all the inclusion criteria and none of the following exclusion criteria: | |
| • Pathologically confirmed adenocarcinoma of the pancreas that has not been previously treated in the metastatic setting | | • Prior treatment of pancreatic cancer in the metastatic setting with surgery, radiotherapy, chemotherapy or investigational therapy (note: placement of biliary stent is allowed) | |
| | | **•** Prior treatment of pancreatic cancer with cytotoxic doses of chemotherapy (patients receiving prior treatment with chemotherapy as a radiation sensitizer are eligible if ≥ 6 months has elapsed from completion of therapy) | |
| | ∘ Part 1: unresectable, locally advanced or metastatic disease is allowed, diagnosed within 6 weeks prior to enrollment | | |
| | | • Known metastasis to the central nervous system | |
| | ∘ Part 2: must have metastatic disease diagnosed within 6 weeks prior to randomization; locally advanced disease is not allowed | • Clinically significant gastrointestinal disorder including hepatic disorders, bleeding, inflammation, occlusion, diarrhea > grade 1, malabsorption syndrome, ulcerative colitis, inflammatory bowel disease, or partial bowel obstruction | |
| | | • History of any second malignancy in the last 3 years; patients with prior history of in-situ cancer or basal or squamous cell skin cancer are eligible. Patients with a history of other malignancies are eligible if they have been continuously disease free for at least 3 years. | |
| • Measurable or non-measurable disease as defined by RECIST v1.1 | | • Known hypersensitivity to any of the components of MM-398, other liposomal products, or any components of 5-FU, leucovorin or oxaliplatin | |
| • ECOG performance status of 0 or 1 | | • Known hypersensitivity to any of the components of nab-paclitaxel or gemcitabine (Part 2 only) | |
| • Adequate biological parameters as evidenced by the following blood counts: | | • Concurrent illnesses that would be a relative contraindication to trial participation such as active cardiac or liver disease, including: | |
| | ∘ ANC > 1,500 cells/µl without the use of hematopoietic growth factors, | | |
| | ∘ Platelet count > 100,000 cells/µl, and | | ∘ Severe arterial thromboembolic events (myocardial infarction, unstable angina pectoris, stroke) less than 6 months before inclusion |
| | ∘ Hemoglobin > 9 g/dL | | |
| • Adequate hepatic function as evidenced by: | | | |
| | | | ∘ NYHA Class III or IV congestive heart failure, ventricular arrhythmias or uncontrolled blood pressure |
| | ∘ Serum total bilirubin ≤ ULN (biliary drainage is allowed for biliary obstruction), and | | |
| | | | ∘ Known historical or active infection with HIV, hepatitis B, or hepatitis C |
| | ∘ AST and ALT ≤ 2.5 x ULN (≤ 5 x ULN is acceptable if liver metastases are present) | • Active infection or an unexplained fever > 38.5°C during screening visits or on the first scheduled day of dosing (at the discretion of the investigator, patients with tumor fever may be enrolled), which in the investigator's opinion might compromise the patient's participation in the trial or affect the study outcome | |
| • Adequate renal function as evidenced by serum creatinine ≤ 1.5 x ULN, and calculated clearance ≥60 mL/min/1.72 m² for patients with serum creatinine levels above or below the institutional normal value. Actual body weight should be used for calculating creatinine clearance using the Cockcroft-Gault Equation (CreatClear = Sex * ((140 - Age) / (SerumCreat)) * (Weight / 72); for patients with body mass index (BMI) >30 kg/m², lean body weight should be used instead. | | | |
| | | • Use of strong CYP3A4 inhibitors or inducers, or presence of any other contraindications for irinotecan | |
| | | • Presence of any contraindications for 5-FU, leucovorin, or oxaliplatin | |
| | | • Use of strong CYP2C8 inhibitors or inducers, or presence of any other contraindications for nab-paclitaxel or gemcitabine (Part 2 only) | |
| | | • Any other medical or social condition deemed by the Investigator to be likely to interfere with a patient's ability to sign informed consent, cooperate and participate in the study, or interfere with the interpretation of the results | |
| • Normal ECG or ECG without any clinically significant findings | | • Pregnant or breast feeding; females of child-bearing potential must test negative for pregnancy at the time of enrollment based on a urine or serum pregnancy test. Both male and female patients of reproductive potential must agree to use a highly effective method of birth control, during the study and for 3 months following the last dose of study drug. | |
| • Recovered from the effects of any prior surgery or radiotherapy | | | |
| • ≥ 18 years of age | | | |
| • Agreeable to submit unstained archived tumor tissue for analysis, if available | | | |
| • Able to understand and sign an informed consent (or have a legal representative who is able to do so) | | | |

### Dose Modifications

The toxicity of each cycle must be recorded prior to the administration of a subsequent cycle and graded according to the National Cancer Institute Common Terminology Criteria for Adverse Events (NCI CTCAE) (Version 4.03). All dose reductions for all arms should be based on the worst preceding toxicity.

Dosing may be held for up to 2 weeks from when it was due to allow for recovery from toxicity related to the study treatment. If the time required for recovery from toxicity is more than 2 weeks, the patient should be discontinued from the study, unless the patient is benefiting from the study treatment, in which case the patient's continuation on study should be discussed between Investigator and Sponsor regarding risks and benefits of continuation. If oxaliplatin is not well tolerated in patients enrolled in Arm 1, oxaliplatin may be discontinued and patients may continue to receive MM-398 + 5-FU/LV at the discretion of the Investigator.

If a patient's dose is reduced during the study due to toxicity, it should remain reduced for the duration of the study; dose re-escalation to an earlier dose is not permitted. Any patient who has 2 dose reductions and experiences an adverse event that would require a third dose reduction must be discontinued from study treatment.

### Dose Modifications

Prior to each dosing, patients must have: ANC ≥ 1500/mm³, WBC ≥ 3500/ mm³, Platelet count ≥ 100,000/mm³ and Diarrhea ≤ Grade 1.

Treatment should be delayed to allow sufficient time for recovery to levels noted above, and upon recovery, treatment should be administered according to the guidelines in the tables below. If the patient had febrile neutropenia, the ANC must have resolved to ≥ 1500/mm³ and the patient must have recovered from infection. For Grade 3 or 4 non-hematological toxicities, treatment should be delayed until they resolve to Grade 1 or baseline. Guidelines for dose adjustments of each individual treatment within the regimen are found in the tables below for Arm 1 (Table 3), and for Arm 2 (Tables 6 through 14). In case a patient experiences an infusion reaction, either institutional guidelines or the guidelines provided for infusion reaction management should be followed.

For all tables below, patient should be withdrawn from study treatment if more than 2 dose reductions are required or if MM-398 reductions lower than 35 mg/m²are required. No dose adjustments for toxicity are required for leucovorin. Leucovorin must be given immediately prior to each 5-FU dose; hence, if 5-FU dose is held, leucovorin dose should be held as well.

Treatment discontinuation that is required due to MM-398 or 5-FU toxicity will result in discontinuation from the study. However, for Arm 1, toxicity that requires discontinuation from oxaliplatin only (e.g. neuropathy) will result in the option to continue on study treatment with MM-398 + 5-FU/LV only for all future dosing.

### Arm 1 Dose Modifications

The starting dose of ONIVYDE will be 60mg/m², 5FU 2400mg/m², LV 400mg/m² and Oxaliplatin either 85mg/m² or 60mg/ m². Dose reduction will be 25% reduction in all agents for any grade III-IV Hematotoxicity. For persistent toxicities despite the first dose reduction, and additional 25% dose reduction in all agents will occur. Further toxicity will then lead to discontinuation from trial.

For non-hematologic toxicities, the dose reduction will be the same dose reduction schema as for hematotoxicity, except for the specific toxicities associated with the drug (ie 5FU hand foot syndrome, and oxaliplatin neuropathy) which will be as shown in Table 3.

**Table 3: Arm 1 Dose Modifications**

| **Worst Toxicity by CTCAE Grade** | **MM-398** | **5-FU** | **Oxaliplatin** |
|---|---|---|---|
| **Hematological Toxicities** | | | |
| Grade 2 neutropenia (ANC <1500 - 1000 cells/ mm³) | 100 % of previous dose | 100 % of previous dose | 1^{st} occurrence: 100% of previous dose |
| Grade 3 or 4 neutropenia (ANC ≤ 1000/mm³) or febrile neutropenia^{a} | 1^{st} occurrence: Reduce dose to 45 mg/m² | 1^{st} occurrence: Reduce dose by 25% | 1^{st} occurrence: Reduce dose from 85 mg/ m² to 65 mg/m² or from 60 mg/m² to 45 mg/m²2^{nd} occurrence: Reduce dose from 65 mg/ m² to 50 mg/m² or from 45 mg/m² to 35 mg/m² |
| | 2^{nd} occurrence: Reduce dose to 35 mg/m² | 2^{nd} occurrence: Reduce dose another 25% | |
| ≥ Grade 2 thrombocytopenia (Grade 2: platelets ≤ 75,000/mm³-50,000/mm³ OR | If Grade 2: 100% of previous dose | If Grade 2: 100% of previous dose | 1^{st} occurrence: Reduce dose from 85 mg/ m² to 65 mg/m² or from 60 mg/m² to 45 mg/m² |
| | If ≥ Grade 3: | If ≥ Grade 3: | |
| | 1^{st} occurrence: Reduce dose to 45 mg/m² | 1^{st} occurrence: Reduce dose by 25% | |
| | | 2^{nd} occurrence: Reduce dose another 25% (50% of original dose) | 2^{nd} occurrence: Reduce dose from 65 mg/ m² to 50 mg/m² or from 45 mg/m² to 35 mg/m² |
| Grade 3-4: platelets < 50,000/mm³) | 2^{nd} occurrence: Reduce dose to 35 mg/m² | | |
| Other hematologic toxicities not specifically listed above | | | If ≤ Grade 2: 100% of previous dose |
| | If ≤ Grade 2: 100% of previous dose | If ≤ Grade 2: 100% of previous dose | If ≥ Grade 3: 1^{st} occurrence: Reduce dose from 85 mg/ m² to 65 mg/m² or from 60 mg/m² to 45 mg/m² |
| | If ≥ Grade 3: 1^{st} occurrence: Reduce dose to 45 mg/m² | If ≥ Grade 3: 1^{st} occurrence: Reduce dose by 25% | |
| | | 2^{nd} occurrence: Reduce dose another 25% | |
| | 2^{nd} occurrence: Reduce dose to 35 mg/m² | | 2^{nd} occurrence: Reduce dose from 65 mg/ m² to 50 mg/m² or from 45 mg/m² to 35 mg/m² |

| **Non-Hematological Toxicities Other than Asthenia and Grade 3 Anorexia^{b}** | | | |
|---|---|---|---|
| Grade 1 or 2, including diarrhea^{c} | 100 % of previous dose | 100% of previous dose, except for Grade 2 hand foot syndrome, Grade 2 cardiac toxicity, or any grade neurocerebellar toxicity | 100 % of previous dose |
| Grade 3 or 4, including diarrhea^{d} (except nausea and vomiting) | 1^{st} occurrence: | 1^{st} occurrence: Reduce dose by 25% | 1^{st} occurrence: Reduce dose from 85 mg/ m² to 65 mg/m² or from 60 mg/m² to 45 mg/m² |
| | Reduce dose to 45 mg/m² | 2^{nd} occurrence: Reduce dose another 25% *except for Grade 3 or 4 hand foot syndrome | |
| | 2^{nd} occurrence: Reduce dose to 35 mg/m² | | 2^{nd} occurrence: Reduce dose from 65 mg/ m² to 50 mg/m² or from 45 mg/m² to 35 mg/m² |
| Grade 3 or 4 nausea and/or vomiting despite anti-emetic therapy | Optimize anti-emetic therapy AND | Optimize anti-emetic therapy AND reduce dose by 25% ; if the patient is already receiving a reduced dose, reduce dose an additional 25% | 1^{st} occurrence: Reduce dose from 85 mg/ m² to 65 mg/m² or from 60 mg/m² to 45 mg/m² |
| | 1^{st} occurrence: Reduce dose to 45 mg/m² | | |
| | | | 2^{nd} occurrence: Reduce dose from 65 mg/ m² to 50 mg/m² or from 45 mg/m² to 35 mg/m² |
| | 2^{nd} occurrence: Reduce dose to 35 mg/m² | | |
| Grade 2 hand foot syndrome | 100 % of previous dose^{d} | 1^{st} occurrence: Reduce dose by 25% | 100 % of previous dose |
| | | 2^{nd} occurrence: Reduce dose another 25% | |
| Grade 3 or 4 hand foot syndrome | 1^{st} occurrence: Reduce dose to 45 mg/m² | Discontinue therapy | No dose modifications required |
| | 2^{nd} occurrence: Reduce dose to 35 mg/m² | | |
| Any grade neurocerebellar or ≥ Grade 2 cardiac toxicity | No dose modifications required^{e} | Discontinue therapy | No dose modifications required |
| | | | Grade 2, persistent: Reduce dose from 85 mg/m² to 60 mg/m² or from 60 mg/m² to 45 mg/m² |
| Sensory neuropathy | No dose modifications required^{e} | No dose modifications required^{e} | Grade 3, recovers prior to next cycle: Reduce dose from 85 mg/m² to 60 mg/m² or from 60 mg/m² to 45 mg/m² |
| | | | Grade 3, persistent: Discontinue therapy |
| | | | Grade 4: Discontinue therapy |

| | | | |
|---|---|---|---|
| ^{a}Consider the use of G-CSF for patients who experience ≥ Grade 3 neutropenia or febrile neutropenia. ^{b}Asthenia and Grade 3 Anorexia do not require dose modification ^{c}Grade 1 diarrhea: 2-3 stools/day > pretreatment; Grade 2 diarrhea: 4-6 stools/day > pretreatment ^{d} Grade 3 diarrhea: 7-9 stools/day > pretreatment; Grade 4 diarrhea: > 10 stools/day > pretreatment | | | |

### Arm 2 Dose Modifications

Dosing may be held for up to 3 weeks from when it was due, to allow for recovery from toxicity related to the study treatments. If the time required for recovery from toxicity is more than 3 weeks, the patient should be discontinued from the study, unless the patient is benefiting from the study treatment, in which case the patient's continuation on study should be discussed between Investigator and Sponsor or its designee regarding risks and benefits of continuation.

If a patient's dose is reduced during the study due to toxicity, it should remain reduced for the duration of the study; dose re-escalation to an earlier dose is not permitted. Any patient who has 2 dose reductions and experiences an adverse event that would require a third dose reduction must be discontinued from study treatment.

Infusion reactions will be monitored. Infusion reactions will be defined according to the National Cancer Institute CTCAE (Version 4.0) definition of an allergic reaction/infusion reaction and anaphylaxis, as defined below:

**Table 4**

| |
|---|
| **Grade 1:** Transient flushing or rash, drug fever <38° C (<100.4º F); intervention not indicated |
| **Grade 2:** Intervention or infusion interruption indicated; responds promptly to symptomatic treatment (e.g., antihistamines, NSAIDS, narcotics); prophylactic medications indicated for <24 hrs |
| **Grade 3:** Symptomatic bronchospasm, with or without urticaria; parenteral intervention indicated; allergy-related edema/angioedema; hypotension |
| **Grade 4:** Life-threatening consequences; urgent intervention indicated |

Study site policies or the following treatment guidelines shall be used for the management of infusion reactions.

**Table 5**

| **Grade 1** | | |
|---|---|---|
| | **•** | Slow infusion rate by 50% |
| | **•** | Monitor patient every 15 minutes for worsening of condition |

| **Grade 2** | | |
|---|---|---|
| | **•** | Stop infusion |
| | **•** | Administer diphenhydramine hydrochloride 50 mg IV, acetaminophen 650 mg orally, and oxygen |
| | **•** | Resume infusion at 50% of the prior rate once infusion reaction has resolved |
| | **•** | Monitor patient every 15 minutes for worsening of condition |
| | **•** | For all subsequent infusions, premedicate with diphenhydramine hydrochloride 25-50 mg IV |

| **Grade 3** | | |
|---|---|---|
| | **•** | Stop infusion and disconnect infusion tubing from patient |
| | **•** | Administer diphenhydramine hydrochloride 50 mg IV, dexamethasone 10 mg IV, bronchodilators for bronchospasm, and other medications or oxygen as medically necessary |
| | • | No further treatment with MM-398 will be permitted |

| **Grade 4** | | |
|---|---|---|
| | **•** | Stop the infusion and disconnect infusion tubing from patient |
| | **•** | Administer epinephrine, bronchodilators or oxygen as indicated for bronchospasm |
| | **•** | Administer diphenhydramine hydrochloride 50 mg IV, dexamethasone 10 mg IV |
| | **•** | Consider hospital admission for observation |
| | **•** | No further treatment with MM-398 will be permitted |

For patients who experience a Grade 1 or Grade 2 infusion reaction, future infusions may be administered at a reduced rate (over 120 minutes), with discretion.

For patients who experience a second grade 1 or 2 infusion reaction, administer dexamethasone 10 mg IV. All subsequent infusions should be premedicated with diphenhydramine hydrochloride 50 mg IV, dexamethasone 10 mg IV, and acetaminophen 650 mg orally.

### MM-398 Dose Modifications for Hematological Toxicities

Prior to initiating a new cycle of therapy, the patients must have:
- ANC ≥ 1500/mm³
- Platelet count ≥ 100,000/mm³
Treatment should be delayed to allow sufficient time for recovery and upon recovery, treatment should be administered according to the guidelines in the tables below. **If** the patient had febrile neutropenia, the ANC must have resolved to ≥ 1500/mm³ and the patient must have recovered from infection.

**Table 6: MM-398 Dose Modifications for Neutrophil Count**

| **ANC: cells/mm³ (Worst CTCAE grade)** | **MM-398 Dose for Next Cycle** | | |
|---|---|---|---|
| | **Arm A: Patients Not Homozygous for UGT1A1*28** | **Arm A: Patients Homozygous for UGT1A1*28** | **Arm C: Patients Homozygous for UGT1A1*28** |
| | | **Arm C: Patients Not Homozygous for UGT1A1*28** | |
| ≥ 1000 to 1999 (Grade 1 or 2) | 100% of previous dose | 100% of previous dose | 100% of previous dose |
| < 1000 (Grade 3/4) or febrile neutropenia | Reduce dose by 20 mg/m² to a minimum dose of 40 mg/m² | Reduce dose to 45 mg/m² for the first occurrence and to 35 mg/m² for the second occurrence | Reduce dose to 45 mg/m² for the first occurrence and to 35 mg/m² for the second occurrence |

**Table 7: MM-398 Dose Modifications for Other Hematologic Toxicity**

| **Worst Toxicity CTCAE Grade** | **MM-398 Dose for Next Cycle** | | |
|---|---|---|---|
| | **Arm A: Patients Not Homozygous for UGT1A1*28** | **Arm A: Patients Homozygous for UGT1A1*28** | **Arm C: Patients Homozygous for UGT1A1*28** |
| | | **Arm C: Patients Not Homozygous for UGT1A1*28** | |
| ≤ Grade 2 | 100% of previous dose | 100% of previous dose | 100% of previous dose |
| Grade 3/4 | Reduce dose by 20 mg/m² to a minimum dose of 40 mg/m² | Reduce dose to 45 mg/m² for the first occurrence and to 35 mg/m² for the second occurrence | Reduce dose to 45 mg/m² for the first occurrence and to 35 mg/m² for the second occurrence |

### MM-398 Dose Modifications for Non-Hematological Toxicities

Treatment should be delayed until diarrhea resolves to ≤ Grade 1, and for other Grade 3 or 4 non-hematological toxicities, until they resolve to Grade 1 or baseline. Guidelines for dose adjustment of MM-398 for drug related diarrhea and other Grade 3 or 4 non-hematological toxicities are provided below. Infusion reactions should be handled as described above.

**Table 8: MM-398 Dose Modifications for Diarrhea**

| **Worst Toxicity CTCAE Grade** | **MM-398 Dose for Next Cycle^{a}** | | |
|---|---|---|---|
| | **Arm A: Patients Not Homozygous for UGT1A1*28** | **Arm A: Patients Homozygous for UGT1A1*28** | **Arm C: Patients Homozygous for UGT1A1*28** |
| | | **Arm C: Patients Not Homozygous for UGT1A1*28** | |
| Grade 1 or 2 (2-3 stools/day > pretreatment or 4-6 stools/day > pretreatment) | 100% of previous dose | 100% of previous dose | 100% of previous dose |
| Grade 3 (7-9 stools/day > pretreatment) or Grade 4 (>10 stools/day > pretreatment) | Reduce dose by 20 mg/m² to a minimum dose of 40 mg/m² | Reduce dose to 45 mg/m² for the first occurrence and to 35 mg/m² for the second occurrence | Reduce dose to 45 mg/m² for the first occurrence and to 35 mg/m² for the second occurrence |

**Table 9: MM-398 Dose Modifications for Non-Hematological Toxicities Other than Diarrhea, Asthenia and Grade 3 Anorexia**

| **Worst Toxicity CTCAE Grade** | **MM-398 Dose for Next Cycle** | | |
|---|---|---|---|
| | **Arm A: Patients Not Homozygous for UGT1A1*28** | **Arm A: Patients Homozygous for UGT1A1*28** | **Arm C: Patients Homozygous for UGT1A1*28** |
| | | **Arm C: Patients Not Homozygous for UGT1A1*28** | |
| Grade 1 or 2 | 100% of previous dose | 100% of previous dose | 100% of previous dose |
| Grade 3 or 4 (except nausea and vomiting) | Reduce dose by 20 mg/m² to a minimum dose of 40 mg/m² | Reduce dose to 45 mg/m² for the first occurrence and to 35 mg/m² for the second occurrence | Reduce dose to 45 mg/m² for the first occurrence and to 35 mg/m² for the second occurrence |
| Grade 3 or 4 nausea and or vomiting despite anti emetic therapy | Optimize anti-emetic therapy AND reduce dose by 20 mg/m² to a minimum dose of 40 mg/m² | Optimize anti-emetic therapy **AND** reduce dose to 40 mg/m² | Optimize anti-emetic therapy **AND** reduce dose to 40 mg/m² |

### 5-FU and Leucovorin Dose Modifications

Guidelines for 5-FU dose modifications are provided below. No dose adjustments for toxicity are required for leucovorin. Leucovorin must be given immediately prior to each 5-FU dose; hence, if 5-FU dose is held, leucovorin dose should be held as well. In case a patient experiences an infusion reaction, either institutional guidelines or the guidelines provided for MM-398 infusion reaction management should be used.

### 5-FU Dose Modifications for Hematological Toxicities

Prior to the next dose in a cycle or prior to initiating a new cycle of therapy, the patients must have:
- ANC ≥ 1500/mm³
- WBC ≥ 3500/mm³
- Platelet count ≥ 75,000/mm³ (according to the European summary of product characteristics for 5-FU, the platelets should have recovered to ≥ 100,000/mm³ prior to initiating therapy)

Treatment should be delayed to allow sufficient time for recovery and upon recovery, treatment should be administered according to the guidelines provided in the table below. The duration of the cycles is fixed at 6 weeks, and if a patient is unable to receive the D8, D15 or D22 dose due to toxicity, the dose will be considered as skipped.

**Table 10: 5-FU Dose Modifications for Hematological Toxicities (Arm B & C)**

| **ANC (cells/mm³)** | | **Platelets (cells/mm³)** | **5-FU Dose for D8, D15, D22^{a}** | **5-FU Dose for Next Cycle^{a}** |
|---|---|---|---|---|
| ≥ 1000 | and | ≥ 50,000 | 100% of previous dose | 100% of previous dose |
| 500 - 999 | Or | <50,000 - 25,000 | Hold; when resolved, reduce dose by 25%^{b} | Reduce dose by 25%^{b} |
| < 500 or febrile neutropenia | Or | < 25,000 or thrombocytopenia with bleeding | Hold dose; when resolved, reduce dose by 25%^{b} | Reduce dose by 25% ^{b} |

| | | | | |
|---|---|---|---|---|
| ^{a} All dose modifications should be based on the worst preceding toxicity ^{b} Patients who require more than 2 dose reductions must be withdrawn from the study | | | | |

### 5-FU Dose Modifications for Non-Hematological Toxicities

Treatment should be delayed until all Grade 3 or 4 non-hematological toxicities resolve to Grade 1 or baseline. Guidelines for dose adjustment of 5-FU related toxicities are provided below. The duration of the cycles is fixed at 6 weeks, and if a patient is unable to receive the D8, D15 or D22 dose due to toxicity, the dose will be considered as skipped.

**Table 11: 5-FU Dose Modifications for Non-Hematological Toxicities Other than Asthenia and Grade 3 Anorexia^{c}**

| **Worst Toxicity CTCAE Grade** | **5-FU Dose for D8, D15, D22^{a}** | **5-FU Dose for Next Cycle^{a}** |
|---|---|---|
| Grade 1 or 2 | 100% of previous dose, except for Grade 2 hand foot syndrome, Grade 2 cardiac toxicity, or any grade neurocerebellar toxicity | 100% of previous dose, except for Grade 2 hand and foot syndrome, Grade 2 cardiac toxicity, or any grade neurocerebellar toxicity |
| Grade 2 hand foot syndrome | Reduce dose by 25%^{b} | Reduce dose by 25%^{b} |
| Any grade neurocerebellar or ≥ Grade 2 cardiac toxicity | Discontinue therapy | Discontinue therapy |
| Grade 3 or 4 | Hold; when resolved, reduce dose by 25% ^{b}, except for Grade 3 or 4 hand foot syndrome | Reduce dose by 25%^{b},except for Grade 3 or 4 hand foot syndrome |
| Grade 3 or 4 hand foot syndrome | Discontinue therapy | Discontinue therapy |

| | | |
|---|---|---|
| ^{a} All dose modifications should be based on the worst preceding toxicity ^{b} Patients who require more than 2 dose reductions must be withdrawn from the study ^{c} Asthenia and Grade 3 Anorexia do not require dose modification | | |

### MM-398 Dose Modifications for UGT1A1*28 Positive Patients (Arms 1 and 2)

Patients are tested for UGT1A1*28 status during screening, however the result of the test is not required prior to the initial dose of MM-398. All patients will begin dosing at 80 mg/m² (salt), however future doses may be reduced for patients who are positive (i.e. homozygous) for UGT1A1*28 7/7 genotype. For Part 1 patients receiving 80 mg/m² (salt) of MM-398: depending on the overall safety profile seen after the first dose, the dose may be reduced to 60 mg/m² (salt) after discussion between the PI, Sponsor and Medical Monitor. Any Part 1 patients who receive a reduced dose during Cycle 1 due to UGT1A1*28 homozygosity will not be evaluable for the cohort and are replaced.

### Arm 3 Dose Modifications

Dose level reductions required due to toxicities related to nab-paclitaxel and gemcitabine should be made following the guidelines outlined in Table 12.

**Table 12: Dose Level Reductions for nab-Paclitaxel and Gemcitabine**

| **Dose Level** | **Nab-paclitaxel (mg/m²)** | **Gemcitabine (mg/m²)** |
|---|---|---|
| Full dose | 125 | 1000 |
| 1^{st} dose reduction | 100 | 800 |
| 2^{nd} dose reduction | 75 | 600 |
| If additional dose reductions required | Discontinue | Discontinue |

Recommended dose modifications for neutropenia and thrombocytopenia are provided in Table 13 and adjustments related to other toxicities are provided in Table 14.

**Table 13: nab-Paclitaxel and Gemcitabine Dose Modifications at the Start of Each Cycle or Within a Cycle for Neutropenia and/or Thrombocytopenia.**

| **Cycle Day** | **ANC (cells/mm³)** | | **Platelet count (cells/mm³)** | **Nab-paclitaxel** / **Gemcitabine** |
|---|---|---|---|---|
| Day 1 | <1500 | OR | < 100,000 | Delay doses until recovery |
| Day 8 | 500 to < 1000 | OR | 50,000 to < 75,000 | Reduce 1 dose level |
| | < 500 | OR | < 50,000 | Withhold doses |

| Day 15: IF day 8 doses were reduced or given without modification: | | | | |
|---|---|---|---|---|
| | 500 to < 1000 | OR | 50,000 to < 75,000 | Reduce 1 dose level from Day 8 |
| | < 500 | OR | < 50,000 | Withhold doses |

| Day 15: IF day 8 doses were withheld: | | | | |
|---|---|---|---|---|
| | ≥ 1000 | OR | ≥ 75,000 | Reduce 1 dose level from Day 1 |
| | 500 to < 1000 | OR | 50,000 to < 75,000 | Reduce 2 dose levels from Day 1 |
| | < 500 | OR | < 50,000 | Withhold doses |

| | | | | |
|---|---|---|---|---|
| ANC = absolute neutrophil count | | | | |

**Table 14: nab-Paclitaxel and Gemcitabine Dose Modifications for Other Adverse Drug Reactions**

| **Adverse Drug Reaction** | **Nab-paclitaxel** | **Gemcitabine** |
|---|---|---|
| **Febrile Neutropenia:** Grade 3 or 4 | Withhold until fever resolves and ANC ≥ 1500; resume at next lower dose level | |
| **Peripheral Neuropathy:** Grade 3 or 4 | Withhold until improves ≤ Grade 1; resume at next dose level | No dose reduction |
| **Cutaneous Toxicity:** Grade 2 or 3 | Reduce to next lower dose level; discontinue treatment if toxicity persists | |
| **Gastrointestinal Toxicity:** Grade 3 mucositis or diarrhea | Withhold until improves to ≤ Grade 1; resume at next dose level | |

### Disease Evaluation

Tumor responses are evaluated according to the Response Evaluation Criteria in Solid Tumors (RECIST) version 1.1, to establish disease progression by CT or MRI. In addition, other imaging procedures, as deemed appropriate by the Investigator, are performed to assess sites of neoplastic involvement. The same method of assessment must be used throughout the study. Investigators should select target and non-target lesions in accordance with RECIST v1.1 guidelines. Follow up measurements and overall response should also be in accordance with these guidelines.

Tumor assessments should be completed until it has been determined that the patient has progressive disease (in accordance with RECIST v1.1). For patients who do not have documented disease progression per RECIST v. 1.1 at the time of treatment termination, imaging studies should be continually performed into the follow-up period every 8 weeks until disease progression is documented. Continued imaging follow-up on schedule is recommended to reduce potential bias in the evaluations of the impacts of the experimental treatments on disease.

### EORTC-QLQ-C30 and EQ-5D-5L (Part 2 Only)

Health-related quality of life (HRQL) is assessed by the EORTC-QLQ-C30 and EQ-5D-5L instruments. The EORTC-QLQ-C30 is a reliable and valid measure of the quality of life of cancer patients in multicultural clinical research settings. It incorporates nine multi-item scales: five functional scales (physical, role, cognitive, emotional, and social); three symptom scales (fatigue, pain, and nausea and vomiting); and a global health and quality-of-life scale. Several single-item symptom measures are also included. EQ-5D is a generic, preference-based measurement of HRQL. The EQ-5D-5L descriptive system comprises the following 5 dimensions: mobility, self-care, usual activities, pain/discomfort and anxiety/depression. Each dimension has 5 levels: no problems, slight problems, moderate problems, severe problems, and unable to do.

Patients are required to complete both questionnaires at time points outlined in the Schedule of Assessments. On days that the patient is to receive study drug, assessments should be completed prior to study drug administration. Only those patients for whom validated translations of the questionnaires are available will be required to complete the questionnaire.

### Efficacy Analysis

In the assessments of efficacy, each MM-398-containing arm is compared to the control arm. Efficacy comparisons use stratified analyses, incorporating randomization strata. Each comparison uses 0.10 level one-sided testing to evaluate whether the MM-398 -containing arm improves the efficacy parameter. Confidence intervals are presented at two-sided 95% level for descriptive purposes. Hypothesis tests and confidence intervals are not adjusted for multiple comparisons. The primary efficacy comparisons are based on the ITT population, which includes all randomized patients.

Tumor evaluation is measured according to RECIST v1.1. For each patient, progression free survival time is determined as the time from randomization (for patients in Part 1, the reference start time will be date of first study drug) to the first documented radiographical Progression of Disease (PD), per investigator using RECIST 1.1, or death from any cause, whichever comes first. If the progression or death occurs at a time point that is greater than 12 weeks after the non-PD last tumor assessment, then progression-free survival time is censored at the time of the last non-PD tumor assessment.

A primary analysis is conducted when the Week 24 progression-free status for all randomized patients can be determined, anticipated at approximately 24 weeks after the last patient is randomized. A subsequent analysis for PFS and other endpoints is performed when PFS events have occurred in at least 120 (i.e. 80% of randomized patients) patients.

### Primary Efficacy Analysis

In the intention-to-treat (ITT) analysis, a patient is considered to have achieved progression-free survival at 24 weeks if the patient has data to indicate the patient has not progressed at 24 weeks. That is, a patient is considered a responder if there is at least one non-PD assessment, prior to progression or new anticancer therapy, at Week 24 or later.

Patients who do not meet the 24-week progression-free achievement criteria (e.g. patients progressed/died up to Week 24, patients censored prior to Week 24), if progression or death occurs at a time point that is greater than 12 weeks after the non-PD last tumor assessment.

For each arm, the progression-free survival achievement rate at 24 weeks is estimated by the number of patients meeting the 24 week achievement criteria divided by the number of ITT patients in the arm. The rate estimates are presented with corresponding 95% confidence intervals. Each MM-398 containing arm is assessed for increase in rate relative to the control arm using a one-sided Cochran-Mantel-Haenszel test, incorporating randomization stratification factors, at 0.10 level of significance.

### Secondary Efficacy Analyses

Progression-Free Survival (PFS) is descriptively summarized for each arm using Kaplan-Meier methodology. Median PFS time and corresponding 95% confidence limits are presented. For each MM-398-containing arm, PFS is compared to the control arm. Hypothesis tests are conducted for differences in PFS using a one-sided stratified log-rank test. Hazard ratios (with 95% confidence interval) for PFS are estimated using stratified Cox models.

Best Overall Response (BOR) is defined as the best response as recorded from the start of study drug until disease progression. Patients without a post-baseline tumor assessment are considered to be non-evaluable for BOR. To classify BOR as stable disease (SD), there should be a qualifying SD assessment at least 6 weeks from randomization. Objective Response Rate (ORR) is defined as the proportion of patients with a BOR characterized as either a Complete Response (CR) or Partial Response (PR) relative to the total number of evaluable patients. Only patients with measurable disease at baseline will be included in the analysis of the objective response. Estimates of objective response rate and its corresponding 95% CI are calculated for each treatment arm. For each MM-398-containing arm, ORR is compared to the control arm. Differences in objective response rate between each MM-398-containing arm and control arm are provided with 95% Cls. Cochran-Mantel-Haenszel tests, adjusting by randomization strata, are used to compare objective response rates.

The maximum reduction (% change from baseline) in CA19-9 is computed, including analyses by time period (up to Week 8, 16 and 24 visits). CA 19-9 response analyses is carried out using 3 thresholds for maximum reduction: ≥ 20%, ≥50%, ≥90%. A patient without post-baseline CA19-9 measurement is considered as a non-responder. Only patients with CA 19-9 elevated (>37 U/mL) at baseline are included in the analysis of the CA19-9 response. For each threshold and time period, the proportion of CA19-9 response is estimated, along with corresponding 95% confidence intervals, by treatment arm.

Overall Survival (OS) is the time from randomization to the date of death from any cause. Patients who are alive or lost to follow-up at the time of the analysis will be censored at the last known alive date. OS is descriptively summarized for each arm using Kaplan-Meier methodology. For each MM-398-containing arm, OS is compared to the control arm. Hypothesis tests are conducted for differences in OS using a one-sided stratified log-rank test. Hazard ratios (with 95% confidence interval) for PFS are estimated using stratified Cox models.

### Quality of Life Analyses

Quality of life analyses are performed using patients in the analysis populations for each quality of life instrument (EORTC-QLC-C30, EQ-5D-5L). EORTC-QLQ-30 and EQ-5D-5L results will be summarized at each visit by treatment group
For each EORTC QLQ-C30 administered, scores are computed for the following scales: Global Health Status, Physical Functioning, Role Functioning, Emotional Functioning, Cognitive Functioning, Social Functioning, Fatigue, Nausea and vomiting, Pain, Dyspnea, Insomnia, Appetite Loss, Constipation, Diarrhea, Financial difficulties.

Scoring is carried out as described in the EORTC QLQ-C30 Scoring Manual (Fayers, Aaronson, Bjordal, Curran, & Groenvald, 2001). Linear transformations are applied to the raw scores so that the reported score will have range 0-100 for all scales. Summary statistics are presented for each subscale. A summary health state index value is computed for each EQ-5D-5L assessment. Summary statistics are presented for summary health state index. For each EQ-5D-5L attribute (mobility, self-care, usual activities, pain/discomfort, and anxiety/depression), responses are tabulated.

### Safety Analysis

Safety analyses (adverse events and laboratory analyses) will be performed using the safety population. Adverse events are reported by the MedDRA version 17.1 or higher. Toxicity is graded according to the NCI CTCAE version 4.03.

Safety analysis of patients in Part 1 is to include a summary of dose-limiting toxicity events.

The period for treatment-emergent adverse events and safety findings is from the time of first study drug administration to 30 days after the date of last study drug administration. If an adverse event begins on the date of first study drug administration with no time recorded, the event is then considered as treatment-emergent.

Tabular summaries are to be presented for all adverse events, pre-treatment adverse events, treatment-emergent adverse events (TEAE), serious adverse events, adverse events leading to study drug discontinuation, TEAE-related to study drug and TEAE Grade 3/4. Adverse events are to be summarized by System Organ Class and preferred term. All adverse event data is to be listed by patient.

Laboratory data is presented by cycle. Abnormal laboratory values are assessed using all available data and toxicity grading will be assigned according to NCI CTCAE toxicity scale, where criteria are available to do so. Maximum and minimum decrease/increase in continuous laboratory data are reported. Frequency and percent of abnormal laboratory values (L/ULN, 2*L/ULN) are assessed. Shift to most severe toxicity grade are summarized.

Vital signs and ECG are tabulated for the change from baseline by time point. Additional analyses may be performed as described in detail within the SAP.

Vital signs are tabulated for the change from baseline by time point. Additional analyses may be performed as described in detail within the SAP.

### Biomarker Subgroup Analysis

Analyses are performed to assess the associations between potential biomarkers (from plasma and archived tissue) and efficacy parameters (ORR, percent change in target lesion size, and PFS or as appropriate). Graphical displays are performed when appropriate.

### Pharmacokinetics Analysis

Plasma concentrations of MM-398 and oxaliplatin can be used to characterize PK parameters. Due to the sparse PK sampling schedule, PK parameters for individual patients can be estimated based on the Empirical Bayesian Estimation method with priors from the previously estimated (MM-398) or published (oxaliplatin) population PK model parameters. The model simulated exposures, e.g., Cₘₐₓ, AUC (area under the curve), are used to examine any possible interactions between MM-398 and oxaliplatin by comparing the least squares geometric mean ratios (LS-GMR) of drug exposures. NONMEM^{®}, Version 7.3, is used to estimate individual PK parameters and simulate plasma exposures.

### Example 4: Tolerability of Antineoplastic Therapies in Human Clinical Trial

The tolerability of antineoplastic therapies combining liposomal irinotecan, 5-FU/leucovorin and oxaliplatin was evaluated in a human clinical trial described in Example 3, using two different doses: 80 mg/m² (salt) of liposomal irinotecan (MM-398) and 60 mg/m² (salt) of liposomal irinotecan (MM-398). Table 15 summarizes three dosing regimens for the treatment of previously untreated (front-line) pancreatic cancer in humans over a 28 day treatment cycle.

**Table 15 Part 1 Dose Escalation Table (MM-398 + 5-FU/LV + oxaliplatin)**

| **Level** | **Oxaliplatin** | | **5-FU/LV** | | **MM-398 (nal-IRI)** | |
|---|---|---|---|---|---|---|
| | **Dose (mg/m²)^{a}** | **Dose Day^{c}** | **Dose (mg/m²)^{b}** | **Dose Day^{c}** | **Dose (mg/m²)** | **Dose Day^{c}** |
| 1 | 60 | 1, 15 | 2400/400 | 1, 15 | 80 | 1, 15 |
| 2 | 85 | 1, 15 | 2400/400 | 1, 15 | 80 | 1, 15 |
| -2A^{d} | 75 | 1, 15 | 2400/400 | 1, 15 | 80 | 1, 15 |

| | | | | | | |
|---|---|---|---|---|---|---|
| a First dose administration in conjunction with first dose of nal-IRI; oxaliplatin to be administered 2 hours after the completion of the nal-IRI infusion in Part 1. b 46 hour infusion, no bolus is given; leucovorin and 5-FU will be administered last, following the completion of the oxaliplatin infusion c Day indicated is part of a 28-day cycle Note: The dose of nal-IRI and 5-FU/LV in Dose Level 1 and 2 above is the same dose and schedule that was previously used in the NAPOLl-1 Phase 3 study. | | | | | | |

Initially, a combination of oxaliplatin, MM-398 liposomal irinotecan, leucovorin and 5-fluorouracil at dose level 1 in Table 15 above. The results are summarized in Table 16 for dose level 1 in Table 15 above (for 80 mg/m² (salt) M-398 dose), showing that the 80 mg/m² (salt) dose of liposomal irinotecan (MM-398) in combination with oxaliplatin and 5-fluorouracil/leucovorin at dose level 1 was not tolerated in humans.

**Table 16: Antineoplastic Therapy with 80 mg/m² liposomal irinotecan in combination with oxaliplatin/5FU/leucovorin in human clinical trials**

| Patient | Cycle 1 Day 1 | Cycle 1 Day 15 | Cycle 2 Day 1 | Cycle 2 Day 15 | Cycle 3 Day 1 | Cycle 3 Day 15 |
|---|---|---|---|---|---|---|
| 1 | ✔ | ✔ | X | X | X | X |
| 2 | ✔ | R | R | R | X | X |
| 3 | ✔ | X | X | X | X | X |
| 4 | ✔ | ✔ | X | X | X | X |
| 5 | ✔ | X | X | X | X | X |
| 6 | ✔ | ✔ | R | R | R | R |
| 7 | ✔ | X | X | X | X | X |

Table 16 summarizes the results from treating a total of seven (7) patients as part of Part 1 of Arm 1 shown in Figure 12. All seven patients met the applicable inclusion criteria specified below, including a diagnosis of pancreatic cancer.

A "check mark" (✔) in Table 16 indicates the patient received the antineoplastic therapy of dose level 1 in Table 15 above, starting on the indicated days of 3 consecutive 28-day treatment cycles: 80 mg/m² liposomal irinotecan (MM-398, dose based on the corresponding amount of irinotecan hydrochloride trihydrate salt), 60 mg/m² oxaliplatin, 400 mg/m² (l+d) leucovorin and 2,400 mg/m² 5-fluorouracil, as described in the protocol of Example 3.

A "R" in Table 16 indicates the patient received a reduced dose of antineoplastic therapy of dose level -1 in Table 2 (Example 3 above) on the corresponding cycle and day: 60 mg/m² liposomal irinotecan (MM-398, dose based on the corresponding amount of irinotecan hydrochloride trihydrate salt), 60 mg/m² oxaliplatin, 400 mg/m² (I+d) leucovorin and 2,400 mg/m² 5-fluorouracil, as described in the protocol of Example 3.

An "X" in Table 16 indicates the patient did not receive an antineoplastic therapy combining liposomal irinotecan, oxaliplatin, 5-fluorouracil and leucovorin or combining liposomal irinotecan, oxaliplatin, and 5-fluorouracil. After cycle 1, day 1 and prior to cycle 1, day 15, patient 2 was determined to be homozygous for the UGT1A1*28 allele, and subsequent reduced doses of the antineoplastic therapy were administered on days indicated in Table 16, based on the protocol of Example 3. Patients 1 and 3-7 were not homozygous for UGT1A1*28 allele.

The antineoplastic therapy of dose level 1 in Table 15 (Example 4) was only administered to 2 of these 6 patients on day 15 of (28-day) cycle 1, no patients received dose level 1 for more than 2 consecutive doses, and none of the patients received this therapy after cycle 1.

Accordingly, as noted in the Table 16, antineoplastic therapies combining a dose of 80 mg/m² liposomal irinotecan with 60 mg/m² oxaliplatin and doses of 2,400 and 400 mg/m² of 5-fluorouracil and (I+d) leucovorin were not well tolerated in a human clinical trial (resulting in dose limiting toxicities). Examples of antineoplastic therapies combining a dose of 80 mg/m² liposomal irinotecan with 60 mg/m² oxaliplatin and doses of 2,400 and 400 mg/m² of 5-fluorouracil and (I+d) leucovorin include the therapies in Table 15.

In contrast, as noted in Table 18 below, antineoplastic therapies combining a dose of 60 mg/m² liposomal irinotecan with 60 mg/m² oxaliplatin and doses of 2,400 and 400 mg/m² of 5-fluorouracil and (I+d) leucovorin were tolerated in a human clinical trial. In particular, dose level -1 in Table 17 (a 60 mg/m² (salt) M-398 dose) was administered two or more consecutive times to multiple human patients in the clinical trial described in Example 3. These antineoplastic therapies comprising the reduced 60 mg/m² (salt) of liposomal irinotecan (MM-398) in combination with oxaliplatin and 5-fluorouracil/leucovorin were better tolerated in humans than dose level 1 in Table 15. In other embodiments, patients are administered the therapy of dose level -2B in Table 17.

**Table 17 Part 1 Dose Escalation Table (MM-398 + 5-FU/LV + oxaliplatin)**

| **Level** | **Oxaliplatin** | | **5-FU/LV** | | **MM-398 (nal-IRI)** | |
|---|---|---|---|---|---|---|
| | **Dose (mg/m²)^{a}** | **Dose Day^{c}** | **Dose (mg/m²)^{b}** | **Dose Day^{c}** | **Dose (mg/m²)** | **Dose Day^{c}** |
| -1 | 60 | 1, 15 | 2400/400 | 1, 15 | 60 | 1, 15 |
| -2B | 85 | 1, 15 | 2400/400 | 1, 15 | 60 | 1, 15 |

| | | | | | | |
|---|---|---|---|---|---|---|
| a First dose administration in conjunction with first dose of MM-398; oxaliplatin to be administered 2 hours after the completion of the nal-IRI infusion in Part 1. b 46 hour infusion, no bolus is given; leucovorin and 5-FU will be administered last, following the completion of the oxaliplatin infusion c Day indicated is part of a 28-day cycle | | | | | | |

**Table 18: Antineoplastic Therapy with 60 mg/m² liposomal irinotecan in combination with oxaliplatin/5FU/leucovorin in human clinical trials**

| Patient | Cycle 1 Day 1 | Cycle 1 Day 15 | Cycle 2 Day 1 | Cycle 2 Day 15 | Cycle 3 Day 1 |
|---|---|---|---|---|---|
| 1 | ✔ | ✔ | R2 | R2 | R2 |
| 2 | ✔ | ✔ | ✔ | | |
| 3 | ✔ | ✔ | ✔ | | |
| 4 | ✔ | ✔ | | | |
| 5 | ✔ | ✔ | ✔ | | |

Table 18 summarizes the results from treating a total of five (5) patients as part of Part 1 of Arm 1 shown in Figure 12. All five patients met the applicable inclusion criteria specified in Example 3, including a diagnosis of pancreatic cancer. A "check mark" (✔) in Table 18 indicates the patient received the antineoplastic therapy of dose level -1 in Table 17 above, starting on the indicated days of 3 consecutive 28-day treatment cycles: 60 mg/m² liposomal irinotecan (MM-398, dose based on the corresponding amount of irinotecan hydrochloride trihydrate salt), 60 mg/m² oxaliplatin, 400 mg/m² (I+d) leucovorin and 2,400 mg/m² 5-fluorouracil, as described in the protocol of Example 3.

In contrast to the antineoplastic therapy of dose level 1 in Table 14, the antineoplastic therapy of dose level -1 in Table 2 (Example 3) was administered repeatedly to patients 2 and 6 for at least 3 consecutive administrations (including 4 consecutive administrations for patient 6).

The antineoplastic therapy of dose level -1 in Table 2 (Example 3) was administered to 5 of 5 patients on days 1 and 15 of (28-day) cycle 1, and days 1 and 15 of (28 day) to 3 of 4 patients in the study, with no dose limiting toxicities. The antineoplastic therapy of dose level -1 was administered repeatedly to all 5 patients for at least 2 consecutive administrations.

A "check mark" (✔) in Table 18 indicates the patient received the antineoplastic therapy of dose level -1 in Table 17 above, starting on the indicated days of 3 consecutive 28-day treatment cycles: 80 mg/m² liposomal irinotecan (MM-398, dose based on the corresponding amount of irinotecan hydrochloride trihydrate salt), 60 mg/m² oxaliplatin, 400 mg/m² (I+d) leucovorin and 2,400 mg/m² 5-fluorouracil, as described in the protocol of Example 3.

A "R2" in Table 18 indicates the patient received a reduced dose of antineoplastic therapy of dose on the corresponding cycle and day: 50 mg/m² liposomal irinotecan (MM-398, dose based on the corresponding amount of irinotecan hydrochloride trihydrate salt), 60 mg/m² oxaliplatin, 400 mg/m² (l+d) leucovorin and 1,800 mg/m² 5-fluorouracil (a 25% reduction compared to dose level -1 dose), as described in the protocol of Example 3. One patient in Table 18 received this reduced dose in response to Grade II symptoms (non-hematologic), but without a dose limiting toxicity.

Accordingly, as noted in the Table 18, antineoplastic therapies combining a dose of 60 mg/m² liposomal irinotecan with 60 mg/m² oxaliplatin and doses of 2,400 and 400 mg/m² of 5-fluorouracil and (I+d) leucovorin were well tolerated in a human clinical trial. Examples of antineoplastic therapies combining a dose of 80 mg/m² liposomal irinotecan with 60 mg/m² oxaliplatin and doses of 2,400 and 400 mg/m² of 5-fluorouracil and (l+d) leucovorin include the therapies in Table 17.

### Example 5: ONIVYDE^{®} (irinotecan liposome injection) Liposomal Irinotecan

One preferred example of an irinotecan liposome described herein is the product marketed as ONIVYDE^{®} (irinotecan liposome injection). ONIVYDE^{®} is a topoisomerase inhibitor, formulated with irinotecan in a liposomal dispersion, for intravenous use.

The finished ONIVYDE^{®} product is a white to slightly yellow opaque sterile concentrate for infusion. It consists of an isotonic dispersion of liposomes containing irinotecan hydrochloride trihydrate. The liposomes are small unilamellar lipid bilayer vesicles, approximately 110 nm in diameter, enclosing an aqueous compartment that contains irinotecan in a gelated or precipitated state, as sucrosofate salt. The vesicle is composed of 1,2-distearoyl-sn-glycero-3-phosphocholine (DSPC) 6.81 mg/mL, cholesterol 2.22 mg/mL, and methoxy-terminated polyethylene glycol (MW 2000)-distearoylphosphatidyl ethanolamine (MPEG-2000-DSPE) 0.12 mg/mL. Each mL also contains 2-[4-(2-hydroxyethyl) piperazin-1-yl]ethanesulfonic acid (HEPES) as a buffer 4.05 mg/mL and sodium chloride as an isotonicity reagent 8.42 mg/mL. The liposomes are dispersed in an aqueous buffered solution.

The ONIVYDE^{®} product contains irinotecan sucrosofate encapsulated in a liposome, obtained from an irinotecan hydrochloride trihydrate starting material. The chemical name of irinotecan is (S)-4,11-diethyl-3,4,12,14-tetrahydro-4-hydroxy-3,14-dioxo1H-pyrano[3',4':6,7]-indolizino[1,2-b]quinolin-9-yl-[1,4'bipiperidine]-1'-carboxylate. The dosage of ONIVYDE^{®} can be calculated based on the equivalent amount of irinotecan trihydrate hydrochloride starting material used to prepare the irinotecan liposomes, or based on the amount of irinotecan in the liposome. There are about 866 mg of irinotecan per gram of irinotecan trihydrate hydrochloride. For example, an ONIVYDE^{®} dose of 80 mg based on the amount of irinotecan hydrochloride trihydrate starting material actually contains about 0.866x(80mg) of irinotecan in the final product (i.e., a dose of 80 mg/m² of ONIVYDE^{®} based on the weight of irinotecan hydrochloride starting material is clinically equivalent to about 70 mg/m² of irinotecan in the final product). Each 10 mL single-dose vial contains 43 mg irinotecan free base at a concentration of 4.3 mg/mL.

## Claims

1. Liposomal irinotecan for use in a method of treating metastatic adenocarcinoma of the pancreas in a human patient who has not previously received chemotherapy to treat the metastatic adenocarcinoma of the pancreas, wherein the liposomal irinotecan is administered in combination with oxaliplatin, leucovorin, and 5-fluorouracil, the method comprising administering an antineoplastic therapy to the patient, the antineoplastic therapy consisting of:
a. 60 mg/m² of liposomal irinotecan,
b. 60 mg/m² to 85 mg/m² oxaliplatin,
c. 200 mg/m² of (I)-form of leucovorin or 400 mg/m² of the (I+d) racemic form of leucovorin, and
d. 2,400 mg/m² 5-fluorouracil;
wherein the liposomal irinotecan is irinotecan sucrose octasulfate salt liposome injection.

2. Liposomal irinotecan for use according to claim 1, wherein the liposomal irinotecan is administered intravenously, in combination with oxaliplatin, 5-fluorouracil and leucovorin.

3. Liposomal irinotecan for use according to any preceding claim, wherein the liposomal irinotecan is administered, followed by administering the oxaliplatin, followed by administering the leucovorin, followed by administering the 5-fluorouracil.

4. Liposomal irinotecan for use according to any preceding claim, wherein the liposomal irinotecan is administered over 90 minutes, followed by administration of the oxaliplatin over 120 minutes, followed by administration of the leucovorin over 30 minutes, followed by administration of the 5-fluorouracil over 46 hours.

5. Liposomal irinotecan for use according to any preceding claim, wherein the oxaliplatin is administered intravenously over 120 minutes.

6. Liposomal irinotecan for use according to any preceding claim, wherein the leucovorin is administered intravenously over 30 minutes.

7. Liposomal irinotecan for use according to any one of claims 2, 4, 5 or 6, wherein the antineoplastic therapy is administered a total of once every two weeks.

8. Liposomal irinotecan for use according to any preceding claim, wherein the 5-fluorouracil is administered as an infusion over 46 hours.

9. Liposomal irinotecan for use according to any preceding claim, wherein the leucovorin is administered immediately prior to the 5-fluorouracil.

10. Liposomal irinotecan for use according to any preceding claim, wherein the liposomal irinotecan, oxaliplatin and leucovorin is administered on days 1 and 15 of a 28-day treatment cycle.

11. Liposomal irinotecan for use according to any preceding claim, wherein the liposomal irinotecan is administered as an infusion over a total of about 90 minutes.

12. Liposomal irinotecan for use according to any preceding claim, wherein:
i. the liposomal irinotecan is administered intravenously over 90 minutes,
ii. the oxaliplatin is administered intravenously over 120 minutes,
iii. the leucovorin is administered intravenously over 30 minutes, and
iv. the 5-fluorouracil is administered intravenously over 46 hours.

13. Liposomal irinotecan for use according to any preceding claim, wherein the administration of the oxaliplatin begins 2 hours after completing each administration of the liposomal irinotecan.

## Patentansprüche

1. Liposomales Irinotecan zur Verwendung bei einem Verfahren zur Behandlung von metastasiertem Adenokarzinom der Bauchspeicheldrüse bei einem menschlichen Patienten, der zuvor noch keine Chemotherapie zur Behandlung des metastasierten Adenokarzinoms der Bauchspeicheldrüse erhalten hat, wobei das liposomale Irinotecan in Kombination mit Oxaliplatin, Leucovorin und 5-Fluoruracil verabreicht wird, wobei das Verfahren das Verabreichen einer antineoplastischen Therapie an den Patienten umfasst, wobei die antineoplastische Therapie aus Folgendem besteht:
a. 60 mg/m² liposomalem Irinotecan,
b. 60 mg/m² bis 85 mg/m² Oxaliplatin,
c. 200 mg/m² der (l)-Form von Leucovorin oder 400 mg/m² der racemischen (l+d)-Form von Leucovorin und
d. 2400 mg/m² 5-Fluoruracil;
wobei es sich bei dem liposomalen Irinotecan um Irinotecansaccharoseoctasulfatsalz-Liposomeninjektion handelt.

2. Liposomales Irinotecan zur Verwendung gemäß Anspruch 1, wobei das liposomale Irinotecan in Kombination mit Oxaliplatin, 5-Fluoruracil und Leucovorin intravenös verabreicht wird.

3. Liposomales Irinotecan zur Verwendung gemäß einem vorhergehenden Anspruch, wobei das liposomale Irinotecan verabreicht wird, dann das Oxaliplatin verabreicht wird, dann das Leucovorin verabreicht wird und dann das 5-Fluoruracil verabreicht wird.

4. Liposomales Irinotecan zur Verwendung gemäß einem vorhergehenden Anspruch, wobei das liposomale Irinotecan über einen Zeitraum von 90 Minuten verabreicht wird, gefolgt von der Verabreichung des Oxaliplatins über einen Zeitraum von 120 Minuten, gefolgt von der Verabreichung des Leucovorins über einen Zeitraum von 30 Minuten, gefolgt von der Verabreichung des 5-Fluoruracils über einen Zeitraum von 46 Stunden.

5. Liposomales Irinotecan zur Verwendung gemäß einem vorhergehenden Anspruch, wobei das Oxaliplatin über einen Zeitraum von 120 Minuten intravenös verabreicht wird.

6. Liposomales Irinotecan zur Verwendung gemäß einem vorhergehenden Anspruch, wobei das Leucovorin über einen Zeitraum von 30 Minuten intravenös verabreicht wird.

7. Liposomales Irinotecan zur Verwendung gemäß einem der Ansprüche 2, 4, 5 oder 6, wobei die antineoplastische Therapie insgesamt einmal alle zwei Wochen verabreicht wird.

8. Liposomales Irinotecan zur Verwendung gemäß einem vorhergehenden Anspruch, wobei das 5-Fluoruracil als Infusion über einen Zeitraum von 46 Stunden verabreicht wird.

9. Liposomales Irinotecan zur Verwendung gemäß einem vorhergehenden Anspruch, wobei das Leucovorin unmittelbar vor dem 5-Fluoruracil verabreicht wird.

10. Liposomales Irinotecan zur Verwendung gemäß einem vorhergehenden Anspruch, wobei das liposomale Irinotecan, Oxaliplatin und Leucovorin an den Tagen 1 und 15 eines 28-tägigen Behandlungszyklus verabreicht wird.

11. Liposomales Irinotecan zur Verwendung gemäß einem vorhergehenden Anspruch, wobei das liposomale Irinotecan als Infusion über einen Zeitraum von insgesamt etwa 90 Minuten verabreicht wird.

12. Liposomales Irinotecan zur Verwendung gemäß einem vorhergehenden Anspruch, wobei:
i. das liposomale Irinotecan intravenös über einen Zeitraum von 90 Minuten verabreicht wird,
ii. das Oxaliplatin intravenös über einen Zeitraum von 120 Minuten verabreicht wird,
iii. das Leucovorin intravenös über einen Zeitraum von 30 Minuten verabreicht wird und
iv. das 5-Fluoruracil intravenös über einen Zeitraum von 46 Stunden verabreicht wird.

13. Liposomales Irinotecan zur Verwendung gemäß einem vorhergehenden Anspruch, wobei die Verabreichung des Oxaliplatins 2 Stunden nach Abschluss jeder Verabreichung des liposomalen Irinotecans beginnt.

## Revendications

1. Irinotécan liposomal pour une utilisation dans un procédé de traitement de l'adénocarcinome métastatique du pancréas chez un patient humain qui n'a pas encore reçu de chimiothérapie pour traiter l'adénocarcinome métastatique du pancréas, dans lequel l'irinotécan liposomal est administré en combinaison avec de l'oxaliplatine, de la leucovorine et du 5-fluorouracile, le procédé comprenant l'administration d'une thérapie antinéoplasique constituée par :
a. 60 mg/m² d'irinotécan liposomal,
b. 60 mg/m² à 85 mg/m² d'oxaliplatine,
c. 200 mg/m² de la forme (l) de la leucovorine ou 400 mg/m² de la forme racémique (l+d) de la leucovorine, et
d. 2 400 mg/m² de 5-fluorouracile ;
dans lequel l'irinotécan liposomal est une injection de sel d'octasulfate de saccharose irinotécan liposome.

2. Irinotécan liposomal pour une utilisation selon la revendication 1, dans lequel l'irinotécan liposomal est administré par voie intraveineuse, en combinaison avec de l'oxaliplatine, du 5-fluorouracile et de la leucovorine.

3. Irinotécan liposomal pour une utilisation selon une quelconque revendication précédente, dans lequel l'irinotécan liposomal est administré, suivi par l'administration de l'oxaliplatine, suivi de l'administration de la leucovorine, suivi de l'administration du 5-fluorouracile.

4. Irinotécan liposomal pour une utilisation selon une quelconque revendication précédente, dans lequel l'irinotécan liposomal est administré sur 90 minutes, suivi de l'administration de l'oxaliplatine sur 120 minutes, suivi de l'administration de la leucovorine sur 30 minutes, suivi de l'administration du 5-fluorouracile sur 46 heures.

5. Irinotécan liposomal pour une utilisation selon une quelconque revendication précédente, dans lequel l'oxaliplatine est administré par voie intraveineuse sur 120 minutes.

6. Irinotécan liposomal pour une utilisation selon une quelconque revendication précédente, dans lequel la leucovorine est administrée par voie intraveineuse sur 30 minutes.

7. Irinotécan liposomal pour une utilisation selon l'une quelconque des revendications 2, 4, 5 ou 6, dans lequel la thérapie antinéoplasique est administrée au total une fois toutes les deux semaines.

8. Irinotécan liposomal pour une utilisation selon une quelconque revendication précédente, dans lequel le 5-fluorouracile est administré en une perfusion pendant 46 heures.

9. Irinotécan liposomal pour une utilisation selon une quelconque revendication précédente, dans lequel la leucovorine est administrée immédiatement avant le 5-fluorouracile.

10. Irinotécan liposomal pour une utilisation selon une quelconque revendication précédente, dans lequel l'irinotécan liposomal, l'oxaliplatine et la leucovorine sont administrés aux jours 1 et 15 d'un cycle de traitement de 28 jours.

11. Irinotécan liposomal pour une utilisation selon une quelconque revendication précédente, dans lequel l'irinotécan liposomal est administré sous forme d'une perfusion sur un total d'environ 90 minutes.

12. Irinotécan liposomal pour une utilisation selon une quelconque revendication précédente, dans lequel :
i. l'irinotécan liposomal est administré par voie intraveineuse sur 90 minutes,
ii. l'oxaliplatine est administré par voie intraveineuse sur 120 minutes,
iii. la leucovorine est administrée par voie intraveineuse sur 30 minutes et
iv. le 5-fluorouracile est administré par voie intraveineuse sur 46 heures.

13. Irinotécan liposomal pour une utilisation selon une quelconque revendication précédente, dans lequel l'administration de l'oxaliplatine commence 2 heures après l'achèvement de chaque administration de l'irinotécan liposomal.
